# EUROPEAN PATENT APPLICATION

(11) **EP 1 826 201 A1**
(43) Date of publication of application: **29.08.2007**
(21) Application number: 06003724.9
(22) Date of filing: 23.02.2006
(51) Int. Cl.: C07D 211/22, C07D 211/28, A61P 3/10, A61P 15/00, A61K 31/454, A61K 31/4545

(54) **Substituted phenylpiperidine derivatives as melanocortin-4 receptor modulators**

(71) Applicant: Santhera Pharmaceuticals (Schweiz) AG, 4410 Liestal (CH)
(72) Inventor: Soeberdt, Michael, 79618 Rheinfelden (DE); Weyermann, Philipp, 4450 Sissach (CH); von Sprecher, Andreas, 4101 Oberwil (CH); Deppe, Holger, 4052 Basel (CH)
(74) Representative: Grünecker, Kinkeldey, Stockmair & Schwanhäusser Anwaltssozietät

(57) **Abstract**

The present invention relates to substituted phenylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally, all diseases and disorders where the regulation of the MC-4R is involved can be treated with the compounds of the invention.

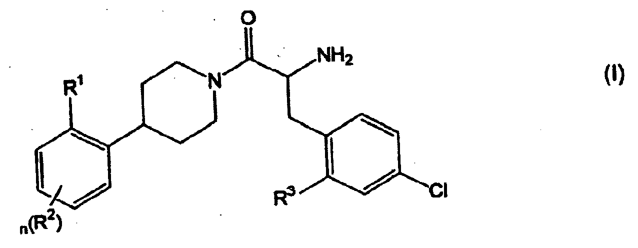

## Description

### Field of the Invention

The present invention relates to substituted phenylpiperidine derivatives as melanocortin-4 receptor modulators. Depending on the structure and the stereochemistry the compounds of the invention are either selective agonists or selective antagonists of the human melanocortin-4 receptor (MC-4R). The agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression. Generally, all diseases and disorders where the regulation of the MC-4R is involved can be treated with the compounds of the invention.

### Background of the Invention

Melanocortins (MCs) stem from pro-opiomelanocortin (POMC) via proteolytic cleavage. These peptides, adrenocorticotropic hormone (ACTH), α-melanocyte-stimulating hormone (α-MSH), β-MSH and γ-MSH, range in size from 12 to 39 amino acids. The most important endogenous agonist for central MC-4R activation appears to be the tridecapeptide α-MSH. Among MCs, it was reported that α-MSH acts as a neurotransmitter or neuromodulator in the brain. MC peptides, particularly α-MSH, have a wide range of effects on biological functions including feeding behavior, pigmentation and exocrine function. The biological effects of α-MSH are mediated by a sub-family of 7-transmembrane G-protein-coupled receptors, termed melanocortin receptors (MC-Rs). Activation of any of these MC-Rs results in stimulation of cAMP formation.

To date, five distinct types of receptor subtype for MC (MC-1 R to MC-5R) have been identified and these are expressed in different tissues.

MC-1R was first found in melanocytes. Naturally occurring inactive variants of MC-1R in animals were shown to lead to alterations in pigmentation and a subsequent lighter coat color by controlling the conversion of phaeomelanin to eumelanin through the control of tyrosinase. From these and other studies, it is evident that MC-1 R is an important regulator of melanin production and coat color in animals and skin color in humans.

The MC-2R is expressed in the adrenal gland representing the ACTH receptor. The MC-2R is not a receptor for α -MSH but is the receptor for the adrenocorticotropic hormone I (ACTH I).

The MC-3R is expressed in the brain (predominately located in the hypothalamus) and peripheral tissues like gut and placenta, and knock-out studies have revealed that the MC-3R may be responsible for alterations in feeding behavior, body weight and thermogenesis.

The MC-4R is primarily expressed in the brain. Overwhelming data support the role of MC-4R in energy homeostasis. Genetic knock-outs and pharmacologic manipulation of MC-4R in animals have shown that agonizing the MC-4R causes weight loss and antagonizing the MC-4R produces weight gain (A. Kask, et al., "Selective antagonist for the melanocortin-4 receptor (HS014) increases food intake in free-feeding rats," Biochem. Biophys. Res. Commun., 245: 90-93 (1998)).

MC-5R is ubiquitously expressed in many peripheral tissues including white fat, placenta and a low level of expression is also observed in the brain. However its expression is greatest in exocrine glands. Genetic knock-out of this receptor in mice results in altered regulation of exocrine gland function, leading to changes in water repulsion and thermoregulation. MC-5R knockout mice also reveal reduced sebaceous gland lipid production (Chen et al., Cell, 91: 789-798 (1997)).

Attention has been focused on the study of MC-3R and MC-4R modulators and their use in treating body weight disorders, such as obesity and anorexia. However, evidence has shown that the MC peptides have potent physiological effects besides their role in regulating pigmentation, feeding behavior and exocrine function. In particular, α-MSH recently has been shown to induce a potent anti-inflammatory effect in both acute and chronic models of inflammation including inflammatory bowel-disease, renal ischemia/reperfusion injury and endotoxin-induced hepatitis. Administration of α-MSH in these models results in substantial reduction of inflammation-mediated tissue damage, a significant decrease in leukocyte infiltration and a dramatic reduction in elevated levels of cytokines and other mediators to near baseline levels. Recent studies have demonstrated that the anti-inflammatory actions of α-MSH are mediated by MC-1 R. The mechanism by which agonism of MC-1 R results in an anti-inflammatory response is likely through inhibition of the pro-inflammatory transcription activator, NF-kB. NF-kB is a pivotal component of the pro-inflammatory cascade, and its activation is a central event in initiating many inflammatory diseases. Additionally, anti-inflammatory actions of α-MSH may be, in part, mediated by agonism of MC-3R and/or MC-5R.

A specific single MC-R that may be targeted for the control of obesity has not yet been identified, although evidence has been presented that MC-4R signaling is important in mediating feeding behavior (S.Q. Giraudo et al., "Feeding effects of hypothalamic injection of melanocortin-4 receptor ligands," Brain Research, 80: 302-306 (1998)). Further evidence for the involvement of MC-Rs in obesity includes: 1) the agouti (A^{vy}) mouse which ectopically expresses an antagonist of the MC-1 R, MC-3R and MC-4R is obese, indicating that blocking the action of these three MC-R's can lead to hyperphagia and metabolic disorders; 2) MC-4R knockout mice (D. Huszar et al., Cell, 88: 131-141 (1997)) recapitulate the phenotype of the agouti mouse and these mice are obese; 3) the cyclic heptapeptide melanotanin II (MT-II) (a non-selective MC-1R, -3R, -4R, and -5R agonist) injected intracerebroventricularly (ICV) in rodents, reduces food intake in several animal feeding models (NPY, ob/ob, agouti, fasted) while ICV injected SHU-9119 (MC-3R and 4R antagonist; MC-1R and -5R agonist) reverses this effect and can induce hyperphagia; 4) chronic intraperitoneal treatment of Zucker fatty rats with an α-NDP-MSH derivative (HP-228) has been reported to activate MC-1R, -3R, -4R, and -5R and to attenuate food intake and body weight gain over a 12 week period (I. Corcos et al., "HP-228 is a potent agonist of melanocortin receptor-4 and significantly attenuates obesity and diabetes in Zucker fatty rats," Society for Neuroscience Abstracts, 23: 673 (1997)).

MC-4R appears to play a role in other physiological functions as well, namely controlling grooming behavior, erection and blood pressure. Erectile dysfunction denotes the medical condition of inability to achieve penile erection sufficient for successful intercourse. The term "impotence" is often employed to describe this prevalent condition. Synthetic melanocortin receptor agonists have been found to initiate erections in men with psychogenic erectile dysfunction (H. Wessells et al., "Synthetic Melanotropic Peptide Initiates Erections in Men With Psychogenic Erectile Dysfunction: Double-Blind, Placebo Controlled Crossover Study," J. Urol., 160: 389-393, 1998). Activation of melanocortin receptors of the brain appears to cause normal stimulation of sexual arousal. Evidence for the involvement of MC-R in male and/or female sexual dysfunction is detailed in WO 00/74679.

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II diabetes" or "Non-Insulin Dependent Diabetes Mellitus" (NIDDM) is the form of diabetes which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle, and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL which are risk factors in cardiovascular diseases. The constellation of symptoms which includes hyperinsulemia combined with hypertension, elevated body weight, elevated triglycerides and elevated LDL, is known as Syndrome X. MC-4R agonists might be useful in the treatment of NIDDM and Syndrome X.

Among MC receptor subtypes, the MC4 receptor is also of interest in terms of the relationship to stress and the regulation of emotional behavior, as based on the following findings. Stress initiates a complex cascade of responses that include endocrine, biochemical and behavioral events. Many of these responses are initiated by release of corticotropin-releasing factor (CRF) (Owen MJ and Nemeroff CB (1991) Physiology and pharmacology of corticotrophin releasing factor. Pharmacol Rev 43: 425-473). In addition to activation of the brain CRF system, there are several lines of evidence that melanocortins (MCs), which stem from proopiomelanocortin by enzymatic processing, mediate important behavioral and biochemical responses to stress and, consequently, stress-induced disorders like anxiety and depression (Anxiolytic-Like and Antidepressant-Like Activities of MCL0129 (1-[(S)-2-(4-Fluorophenyl)-2-(4-isopropylpiperadin-1-yl)ethyl]-4-[4-(2-methoxynaphthalen-1-yl)butyl]piperazine), a Novel and Potent Nonpeptide Antagonist of the Melanocortin-4 Receptor; Shigeyuki Chaki et al, J. Pharm. Exp. Ther. (2003) 304(2), 818-26).

Chronic diseases, such as malignant tumors or infections, are frequently associated with cachexia resulting from a combination of a decrease in appetite and a loss of lean body mass. Extensive loss of lean body mass is often triggered by an inflammatory process and is usually associated with increased plasma levels of cytokines (e.g. TNF-α), which increase the production of α-MSH in the brain. Activation of MC4 receptors in the hypothalamus by α-MSH reduces appetite and increases energy expenditure. Experimental evidence in tumor bearing mice suggests that cachexia can be prevented or reversed by genetic MC4 receptor knockout or MC4 receptor blockade. The increased body weight in the treated mice is attributable to a larger amount of lean body mass, which mainly consists of skeletal muscle (Marks D.L. et al. Role of the central melanocortin system in cachexia. Cancer Res. (2001) 61: 1432-1438).

Modulators of the melanocortin receptor are already known from the literature. WO 03/009847 A1 describes substituted phenylpiperidines derivatives which are considered to be useful in the treatment of obesity.

WO 04/083209 A1 describes substituted piperidine and piperazine derivatives which can be used in the treatment of disorders and diseases responsive to the modulation of the melanocortin-4 receptor.

WO 04/024720 A1 describes piperazine urea derivatives as melanocortin- 4 receptor agonists. The compounds are prepared by the reaction of a 4-substituted piperidine with an N-protected amino acid derivative followed by removal of the amine protecting group to afford an intermediate amine. Said amine is then converted into the desired unsymmetrical piperazine urea compound. No pharmacological acitivity of the intermediate amine is reported.

In view of the unresolved deficiencies in treatment of various diseases and disorders as discussed above, it is an object of the present invention to provide novel substituted phenylpiperidine derivatives with improved ability to cross the blood brain barrier, which are useful as melanocortin-4 receptor modulators to treat cancer cachexia, muscle wasting, anorexia, anxiety, depression, obesity, diabetes, sexual dysfunction and other diseases with MC-4R involvement.

### Summary of the Invention

The present invention relates to substituted phenylpiperidine derivatives of structural formula (I) and structural formula (II) 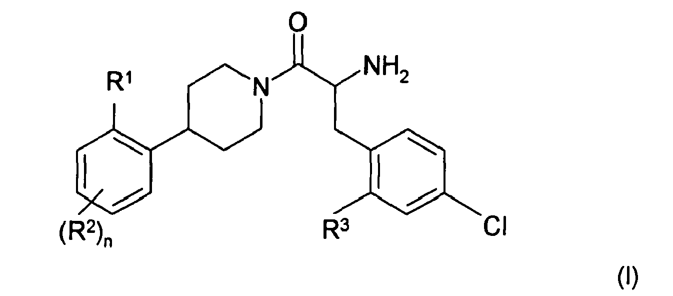 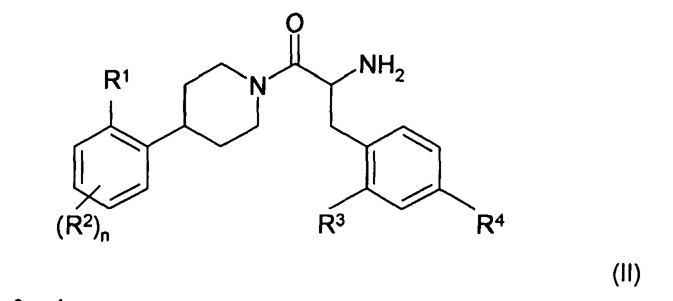 wherein R¹, R², R³, R⁴ and n are defined as described below.

The phenylpiperidine derivatives of structural formulas (I) and (II) are effective as melanocortin receptor modulators and are particularly effective as selective melanocortin-4 receptor (MC-4R) modulators. They are therefore useful for the treatment of disorders where the activation or inactivation of the MC-4R are involved. Agonists can be used for the treatment of disorders and diseases such as obesity, diabetes and sexual dysfunction, whereas the antagonists are useful for the treatment of disorders and diseases such as cancer cachexia, muscle wasting, anorexia, anxiety and depression.

The present invention also relates to pharmaceutical compositions comprising the compounds of the present invention and a pharmaceutically acceptable carrier.

### Detailed Description of the Invention

The present invention relates to substituted phenylpiperidine derivatives useful as melanocortin receptor modulators, in particular, selective MC-4R agonists and MC-4R antagonists.

The compounds of the present invention are represented by structural formula (I) 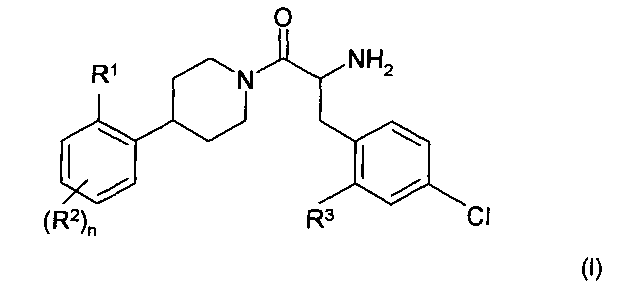 and the enantiomers, diastereomers, tautomers, solvates and pharmaceutically acceptable salts thereof,
wherein
- R¹ is: -(CH₂)_{I}-T,
-O-(CH₂)ₘ-T,
- T is: NR⁵R⁶,
morpholine, 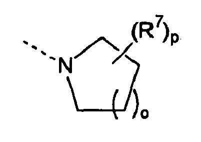 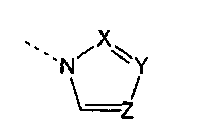
- R⁵ and R⁶ are: independently
C₁₋₆-alkyl,
C₂₋₆-alkenyl,
C₂₋₆-alkinyl,
C₂₋₆-alkylene-O-C₁₋₆-alkyl,
- R⁷ is: halogen,
CN,
OH,
C₁₋₆-alkyl, optionally substituted with 1 to 3 substituents selected from halogen,
CN and OH,
O-C₁₋₆-alkyl, optionally substituted with 1 to 3 substituents selected from halogen, CN and OH,
- X is: CH, N,
- Y is: CH, N,
- Z is: CH, N,
- R² is: F,
Cl,
methyl,
- R³ is: Cl,
methyl,
- I is: 3, 4,
- m is: 2, 3, 4,
- n is: 0, 1, 2, 3, 4,
- o is: 0, 1, 2,
- p is: 0, 1, 2, 3, 4.

Preferably, the compounds according to formula (I) adopt the structural conformation of the following stereoisomer (formula (I'):

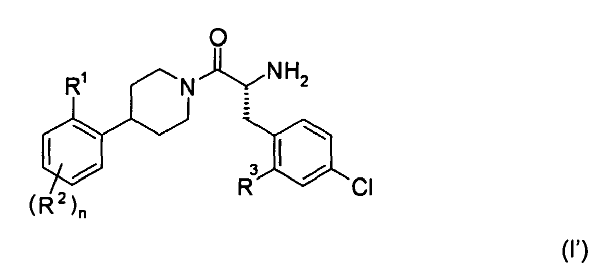

In a preferred embodiment, R² represents Cl. Preferably, the phenyl ring is monosubstituted by a chlorine atom in the meta or para-position. In a further preferred embodiment, the phenyl ring is disubstituted by fluorine atoms.
In a further preferred embodiment, R³ represents Cl.
The variant I is preferably 3.
The variant m is preferably selected from 2 or 3.

The present invention further relates to compounds according to formula (II) 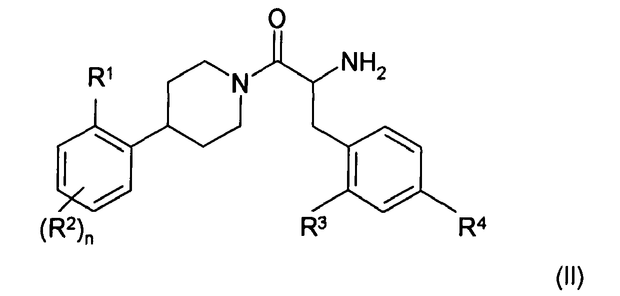 wherein
- R¹ is: -(CH₂)_{I}-T,
-O-(CH₂)ₘ-T,
- T is: NR⁵R⁶,
morpholine, 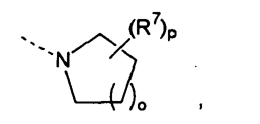 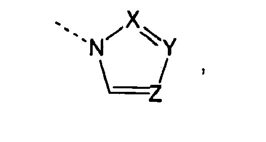
- R⁵ and R⁶ are: independently
C₁₋₆-alkyl,
C₂₋₆-alkenyl,
C₂₋₆-alkinyl,
C₂₋₆-alkylene-O-C₁₋₆-alkyl,
- R⁷ is: halogen,
CN,
OH,
C₁₋₆-alkyl optionally substituted with 1 to 3 substituents selected from halogen,
CN and OH,
O-C₁₋₆-alkyl, optionally substituted with 1 to 3 substituents selected from halogen, CN and OH,
- X is: CH, N,
- Y is: CH, N,
- Z is: CH, N,
- R² is: F,
Cl,
methyl,
- R³ is: H,
F,
- R⁴ is: Cl,
F,
- I is: 3, 4,
- m is: 2, 3, 4,
- n is: 0, 1, 2, 3, 4,
- o is: 0, 1, 2,
- p is: 0, 1, 2, 3, 4.

Preferably, the compounds according to formula (II) adopt the structural conformation of the following stereoisomer (formula (II'):

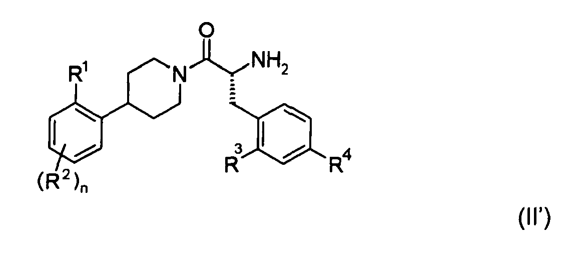

In a preferred embodiment, R² represents Cl and/or F. Preferably, the phenyl ring is monosubstituted by a fluorine atom in the ortho, meta or para-position, most preferably in the para-position. It is further preferred that the phenyl ring is monosubstituted by a chlorine atom in the meta or para-position. In a further preferred embodiment, the phenyl ring is disubstituted by fluorine atoms.
In a further preferred embodiment, R³ represents H.
The variant I is preferably 3.
The variant m is preferably selected from 2 or 3.

As regards compounds of formulas (I) or (II), T is preferably selected from the following radicals:

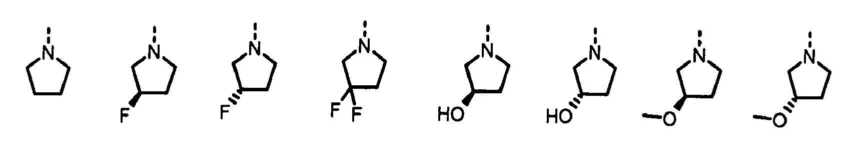

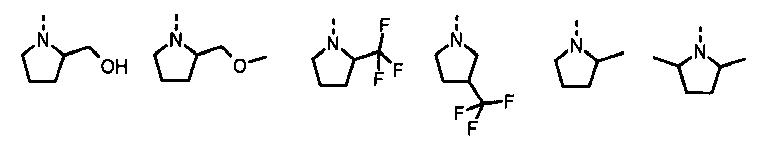

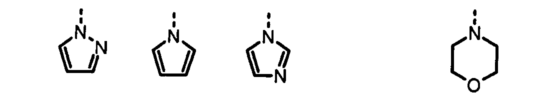

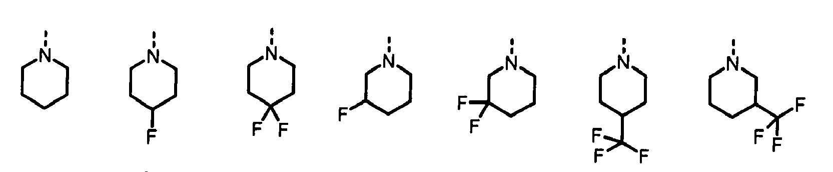

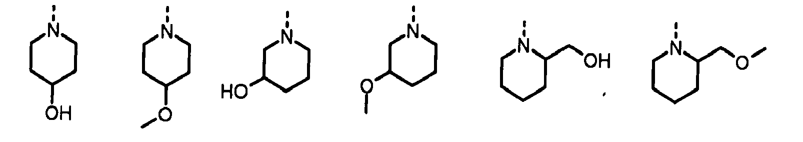

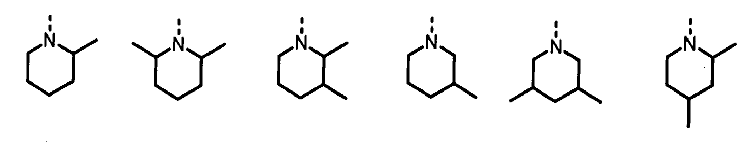

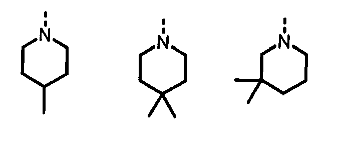

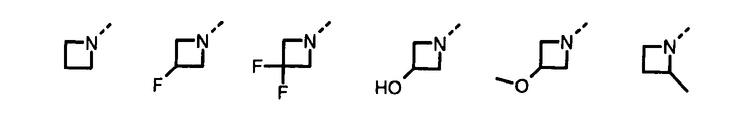

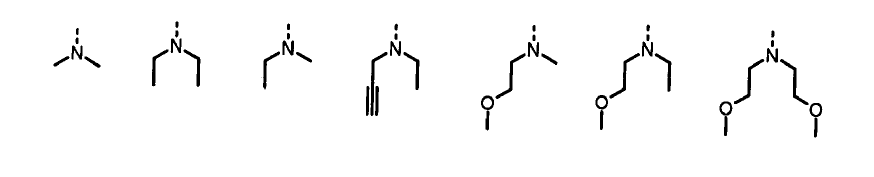

Compounds of the formula (I) or (II) in which some or all of the above-mentioned groups have the preferred or more preferred meanings are also an object of the present invention.

In the above and the following, the employed terms have the meaning as described below:

Alkyl is a straight chain or branched alkyl having 1 to 6 carbon atoms, such as methyl, ethyl, n-propyl, isopropyl, n-butyl, isobutyl, tert-butyl, n-pentyl, isopentyl, neopentyl, or hexyl.

Alkenyl is a straight chain or branched alkyl having 2 to 6 carbon atoms and which contains at least one carbon-carbon double bond, such as vinyl, allyl, 1-propenyl, 2-butenyl, 2-methyl-2-butenyl, isopropenyl, pentenyl, or hexenyl.

Alkinyl is a straight chain or branched alkyl having 2 to 6 carbon atoms and which contains at least one carbon-carbon triple bond, such as ethinyl, 1-propinyl, 1-butinyl, 2-butinyl, pentinyl or hexinyl.

The compounds of structural formulas (I) and (II) are effective as melanocortin receptor modulators and are particularly effective as selective modulators of MC-4R. They are therefore useful for the treatment and/or prevention of disorders responsive to the activation and inactivation of MC-4R, such as cancer cachexia, muscle wasting, anorexia, anxiety, depression, obesity, diabetes, sexual dysfunction and other diseases with MC-4R involvement.

### Optical Isomers - Diastereomers - Geometric Isomers - Tautomers

Compounds of structural formulas (I) and (II) contain one or more asymmetric centers and can occur as racemates and racemic mixtures, single enantiomers, diastereomeric mixtures and individual diastereomers. The present invention is meant to comprehend all such isomeric forms of the compounds of structural formulas (I) and (II).

Compounds of structural formulas (I) and (II) may be separated into their individual diastereoisomers by, for example, fractional crystallization from a suitable solvent, for example methanol or ethyl acetate or a mixture thereof, or via chiral chromatography using an optically active stationary phase. Absolute stereochemistry may be determined by X-ray crystallography of crystalline products or crystalline intermediates which are derivatized, if necessary, with a reagent containing an asymmetric center of known absolute configuration.

Alternatively, any stereoisomer of a compound of the general formulas (I) and (II) may be obtained by stereospecific synthesis using optically pure starting materials or reagents of known absolute configuration.

### Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, fumaric, gluconic, glutamic, hydrobromic, hydrochloric, isethionic, lactic, maleic, malic, mandelic, methanesulfonic, malonic, mucic, nitric, pamoic, pantothenic, phosphoric, propionic, succinic, sulfuric, tartaric, p-toluenesulfonic, trifluoroacetic acid and the like.

Particularly preferred are citric, fumaric, hydrobromic, hydrochloric, maleic, phosphoric, sulfuric and tartaric acids.

It will be understood that, as used herein, references to the compounds of formulas (I) and (II) are meant to also include the pharmaceutically acceptable salts.

### Utility

Compounds of formula (I) are melanocortin receptor antagonists and as such are useful in the treatment, control or prevention of diseases, disorders or conditions responsive to the inactivation of one or more of the melanocortin receptors including, but not limited to, MC-1R, MC-2R, MC-3R, MC-4R or MC-5R. Such diseases, disorders or conditions include, but are not limited to, cancer cachexia, muscle wasting, anorexia, anxiety and depression.

Compounds of formula (II) are melanocortin receptor agonists and as such are useful in the treatment, control or prevention of diseases, disorders or conditions responsive to the activation of one or more of the melanocortin receptors including, but not limited to, MC-1R, MC-2R, MC-3R, MC-4R or MC-5R. Such diseases, disorders or conditions include, but are not limited to, obesity (by reducing appetite, increasing metabolic rate, reducing fat intake or reducing carbohydrate craving), diabetes mellitus (by enhancing glucose tolerance, decreasing insulin resistance) and male and female sexual dysfunction (including impotence, loss of libido and erectile dysfunction).

The compounds of formulas (I) and (II) can be further used in the treatment, control or prevention of diseases, disorders or conditions which are responsive to the activation or inactivation of one or more melanocortin receptors including, but not limited to, MC-1R, MC-2R, MC-3R, MC-4R or MC-5R. Such diseases, disorders or conditions include, but are not limited to, hypertension, hyperlipidemia, osteoarthritis, cancer, gall bladder disease, sleep apnea, compulsion, neuroses, insomnia/sleep disorder, substance abuse, pain, fever, inflammation, immune-modulation, rheumatoid arthritis, skin tanning, acne and other skin disorders, neuroprotective and cognitive and memory enhancement including the treatment of Alzheimer's disease.

### Administration and Dose Ranges

Any suitable route of administration may be employed for providing a mammal, especially a human with an effective dosage of a compound of the present invention. For example, oral, rectal, topical, parenteral, ocular, pulmonary, nasal and the like may be employed. Dosage forms include tablets, troches, dispersions, suspensions, solutions, capsules, creams, ointments, aerosols and the like. Preferably compounds of formulas (I) and (II) are administered orally or topically.

The effective dosage of active ingredient employed may vary depending on the particular compound employed, the mode of administration, the condition being treated and the severity of the condition being treated. Such dosage may be ascertained readily by a person skilled in the art.

When treating cancer cachexia, muscle wasting or anorexia generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating obesity, in conjunction with diabetes and/or hyperglycemia, or alone, generally satisfactory results are obtained when the compounds of the present invention are administered at a daily dosage of from about 0.001 milligram to about 100 milligrams per kilogram of body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

When treating diabetes mellitus and/or hyperglycemia, as well as other diseases or disorders for which compounds of formulas (I) and (II) are useful, generally satisfactory results are obtained when the compounds of the present invention are administered at a daily dosage of from about 0.001 milligram to about 100 milligram per kilogram of animal body weight, preferably given in a single dose or in divided doses two to six times a day, or in sustained release form. In the case of a 70 kg adult human, the total daily dose will generally be from about 0.07 milligrams to about 3500 milligrams. This dosage regimen may be adjusted to provide the optimal therapeutic response.

For the treatment of sexual dysfunction, compounds of the present invention are given in a dose range of 0.001 milligram to about 100 milligram per kilogram of body weight, preferably as a single dose orally or as a nasal spray.

### Formulation

The compounds of formulas (I) and (II) are preferably formulated into a dosage form prior to administration. Accordingly the present invention also includes a pharmaceutical composition comprising a compound of formulas (I) or (II) and a suitable pharmaceutical carrier.

The present pharmaceutical compositions are prepared by known procedures using well-known and readily available ingredients. In making the formulations of the present invention, the active ingredient (a compound of formulas (I) or (II)) is usually mixed with a carrier, or diluted by a carrier, or enclosed within a carrier, which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders.

Some examples of suitable carriers, excipients and diluents include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient.

### Preparation of Compounds of the Invention

When describing the preparation of the present compounds of formulas (I) and (II), the terms "A moiety" and "B moiety" are used below. This moiety concept is illustrated below:

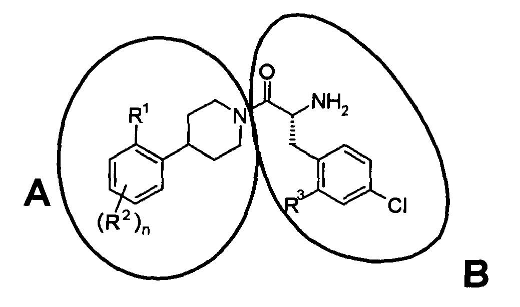

The skilled artisan will recognize that, in general, the two moieties of a compound of formulas (I) and (II) are connected via an amide bond. The skilled artist can, therefore, readily envision numerous routes and methods of connecting the two moieties via standard peptide coupling reaction conditions.

The phrase "standard peptide coupling reaction conditions" means coupling a carboxylic acid with an amine using an acid activating agent such as EDCI, dicyclohexylcarbodiimide and benzotriazol-1-yloxytris(dimethylamino)-phosphonium hexafluorophosphate, in a inert solvent such as DCM, in the presence of a catalyst such as HOBt. The uses of protective groups for amine and carboxylic acids to facilitate the desired reaction and minimize undesired reactions are well documented. Conditions required to remove protecting groups which may be present can be found in Greene, et al., Protective Groups in Organic Synthesis, John Wiley & Sons, Inc., New York, NY 1991.

Protecting groups like Z, Boc and Fmoc are used extensively in the synthesis, and their removal conditions are well known to those skilled in the art. For example, removal of Z groups can he achieved by catalytic hydrogenation with hydrogen in the presence of a noble metal or its oxide, such as palladium on activated carbon in a protic solvent, such as ethanol. In cases where catalytic hydrogenation is contraindicated by the presence of other potentially reactive functionality, removal of Z can also be achieved by treatment with a solution of hydrogen bromide in acetic acid, or by treatment with a mixture of TFA and dimethylsulfide. Removal of Boc protecting groups is carried out in a solvent such as methylene chloride, methanol or ethyl acetate with a strong acid, such as TFA or HCl or hydrogen chloride gas.

The compounds of formulas (I) and (II), when existing as a diastereomeric mixture, may be separated into diastereomeric pairs of enantiomers by fractional crystallization from a suitable solvent such as methanol, ethyl acetate or a mixture thereof. The pair of enantiomers thus obtained may be separated into individual stereoisomers by conventional means by using an optically active acid as a resolving agent. Alternatively, any enantiomer of a compound of the formulas (I) and (II) may be obtained by stereospecific synthesis using optically pure starting materials or reagents of known configuration.

The compounds of formulas (I) and (II) of the present invention can be prepared according to the procedures of the following Schemes and Examples, using appropriate materials and are further exemplified by the following specific examples. Moreover, by utilizing the procedures described herein, in conjunction with ordinary skills in the art, additional compounds of the present invention claimed herein can be readily prepared. The compounds illustrated in the examples are not, however, to be construed as forming the only genus that is considered as the invention. The Examples further illustrate details for the preparation of the compounds of the present invention. Those skilled in the art will readily understand that known variations of the conditions and processes of the following preparative procedures can be used to prepare these compounds. The instant compounds are generally isolated in the form of their pharmaceutically acceptable salts, such as those described previously. The free amine bases corresponding to the isolated salts can be generated by neutralization with a suitable base, such as aqueous sodium hydrogencarbonate, sodium carbonate, sodium hydroxide and potassium hydroxide, and extraction of the liberated amine free base into an organic solvent followed by evaporation. The amine free base isolated in this manner can be further converted into another pharmaceutically acceptable salt by dissolution in an organic solvent followed by addition of the appropriate acid and subsequent evaporation, precipitation or crystallization. All temperatures are degrees Celsius.

In the schemes, preparations and examples below, various reagent symbols and abbreviations have the following meanings:
- AcOH: acetic acid
- Boc: tert-butoxycarbonyl
- Bz₂O₂: dibenzoylperoxide
- DCM: dichloromethane
- DEAD: diethyl azodicarboxylate
- DIAD: diisopropyl azodicarboxylate
- DIEA: ethyl-diisopropylamine
- DMAP: 4-dimethylaminopyridine
- DMF: N,N-dimethylformamide
- dppf: 1,1'-bis(diphenylphosphino)-ferrocen
- EDCI: 1-(3-dimethylaminopropyl)-3-ethylcarbodiimide hydrochloride
- Et₂O: diethyl ether
- EtOH: ethanol
- HOBt: 1-hydroxybenzotriazole
- h: hour(s)
- MeCN: acetonitrile
- MeOH: methanol
- NBS: N-bromosuccinimide
- NMM: N-methylmorpholine
- PPh₃: triphenylphosphine
- THF: tetrahydrofurane

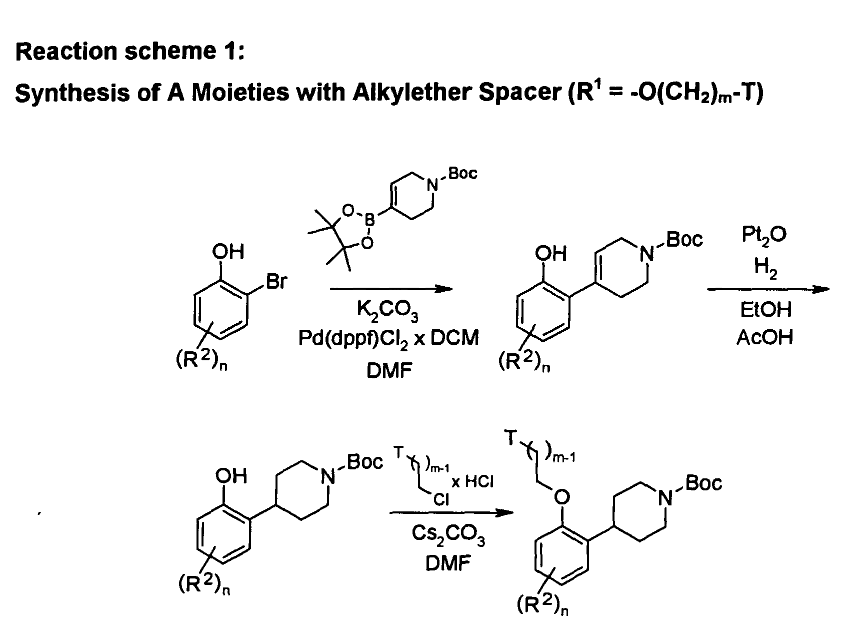

As shown in Reaction Scheme 1 optionally substituted 2-bromo-phenol and 4-(4,4,5,5-tetramethyl-[1,3,2]dioxaborolan-2-yl)-3,6-dihydro-2H-pyridine-1-carboxylic acid tert-butyl ester *(*Tetrahedron Lett. 2000, 41, 3705-3708) are reacted in a Suzuki coupling in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature. The resulting tetrahydropyridine can be hydrogenated in the presence of a catalyst, such as PtO₂ or Pd/C, to yield the protected piperidine. The piperidine is further reacted with an alkylchloride or alkylbromide bearing the capping group T in the presence of a base such as Cs₂CO₃ or NaH in an appropriate solvent such as DMF to give the Boc-protected A moiety.

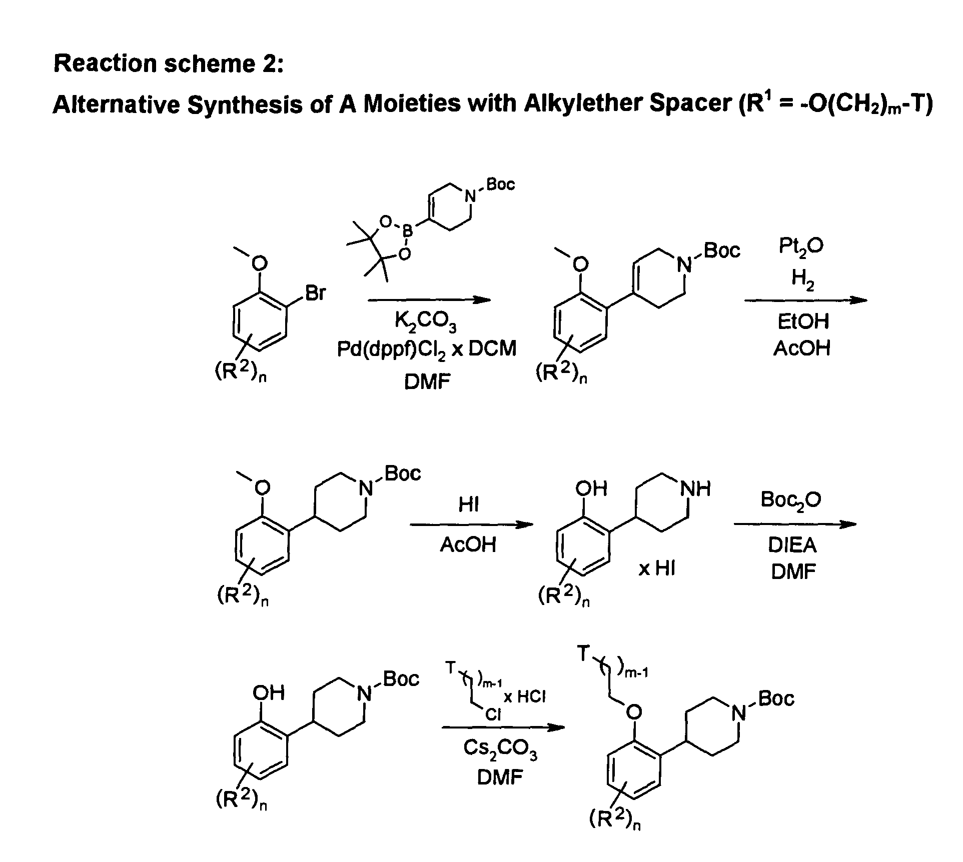

The synthesis of A Moieties bearing an alkylether spacer (R¹ = -O(CH₂)ₘ-T) can alternatively be performed starting from optionally substituted 2-bromoanisole (see Reaction scheme 2). A Suzuki coupling with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature leads to the corresponding tetrahydropyridine. The resulting tetrahydropyridine can be hydrogenated in the presence of a catalyst, such as PtO₂ or Pd/C, to yield the protected piperidine. The methylether can be cleaved with a reagent such as aqueous hydroiodic acid in acetic acid or trimethylsilyl iodide in chloroform, at a suitable temperature to get access to the corresponding phenol as hydroiodide. The Bocprotecting group, which is lost during this process, can subsequently be reintroduced by using a reagent such as Boc₂O in the presence of a base such as DIEA in an appropriate solvent such as DCM. The Boc-protected piperidine is further reacted with an alkylchloride or alkylbromide bearing the capping group T in the presence of a base such as Cs₂CO₃ or NaH in an appropriate solvent such as DMF to give the Boc-protected A moiety.

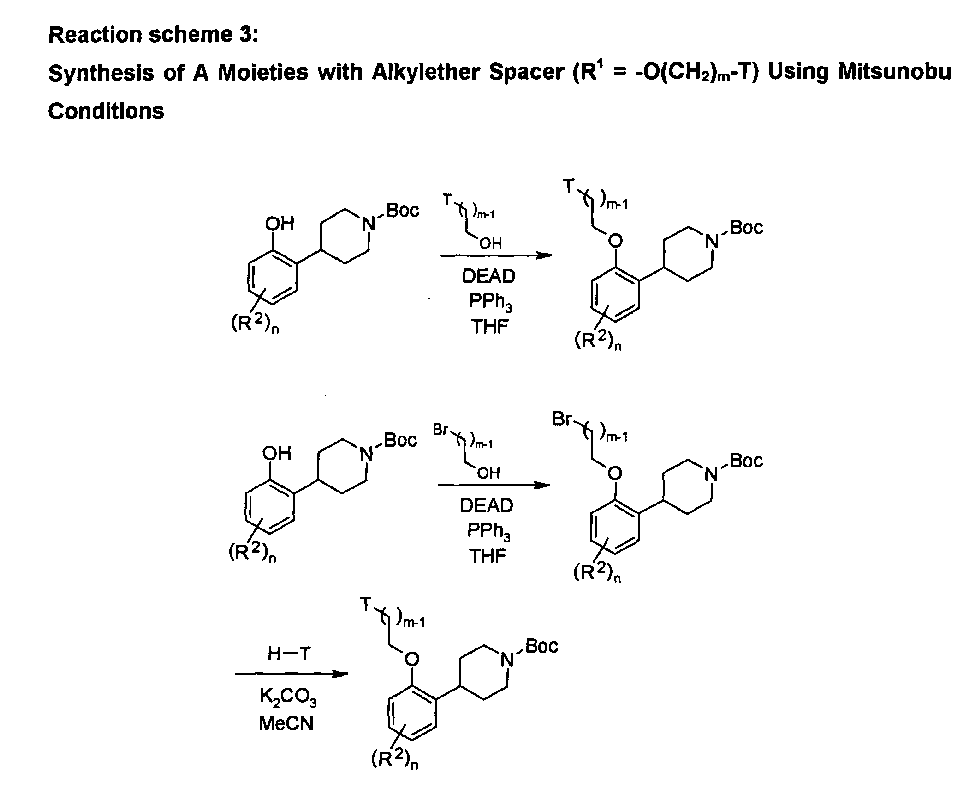

As shown in Reaction scheme 3, the intermediate product from Reaction schemes 1 and 2, optionally substituted 1-Boc-4-(2-hydroxy-phenyl)-piperidine, can also be alkylated with an ω-T-capped alkylalcohol in the presence of a reagent such as DEAD or DIAD and a phosphine such as PPh₃ in a suitable solvent such as THF to give the Boc-protected A moieties.

Similarly, the same intermediate can be reacted with an ω-bromo alkylalcohol, using the reaction conditions described above, to give access to the corresponding phenolether which subsequently can be used to alkylate the capping group T in the presence of a suitable base such as K₂CO₃ or NaH, in an appropriate solvent such as MeCN, THF, or DMF, at a suitable temperature, to yield the Boc-protected A moieties.

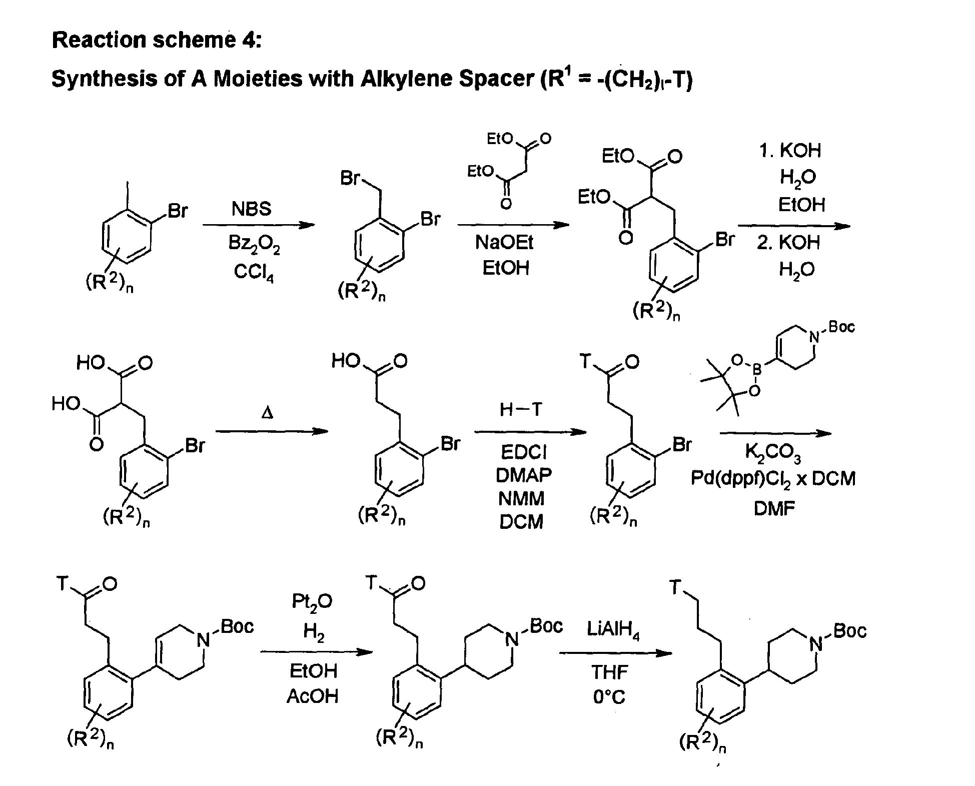

The first route for the synthesis of A moieties bearing an alkylene spacer (R¹ = -(CH₂)₁-T) is depicted in Reaction scheme 4. Optionally substituted 2-bromotoluene is brominated with NBS in the presence of a radical starter such as Bz₂O₂ in an appropriate solvent such as CCl₄ at a suitable temperature to yield the corresponding benzylbromide. The benzylbromide is reacted with diethyl malonate in the presence of a base such as sodium ethoxide in a suitable solvent such as ethanol. Subsequent saponification with a base such as KOH in an appropriate solvent such as water-ethanol mixture followed by a second saponification step with a suitable base such as KOH in a solvent such as water leads to the alkylated malonic acid which is decarboxylated at an appropriate temperature. The product of this reaction, optionally substituted 3-(2-bromophenyl)propionic acid, is activated with a reagent such as EDCI in the presence of a catalyst such as DMAP and a base such as NMM in DCM, and reacted with the capping group T to form the corresponding amide. Optionally substituted 3-(2-bromophenyl)propionic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichloro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

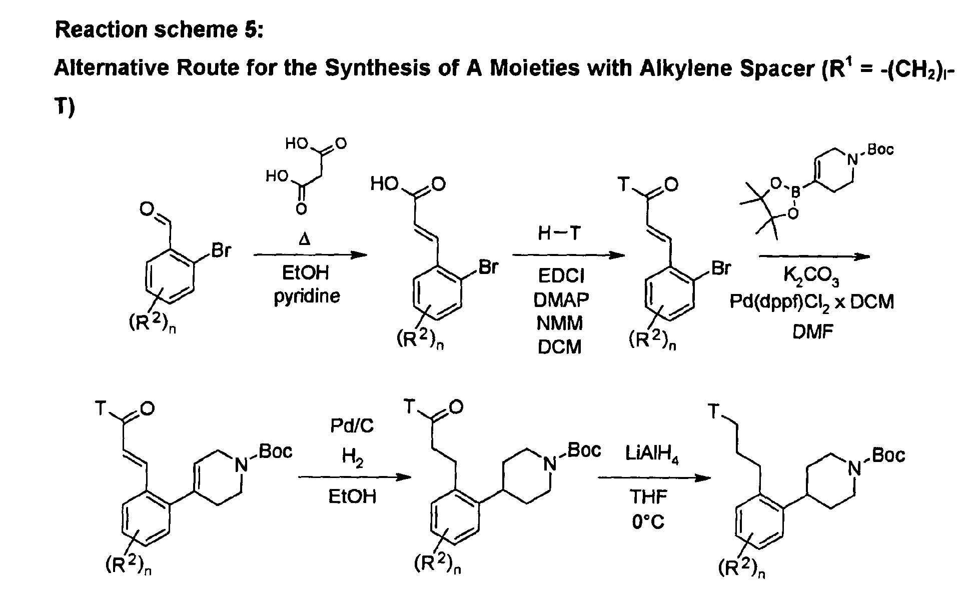

An alternative approach for the synthesis of of A moieties bearing an alkylene spacer (R¹ = -(CH₂)₁-T) starts with optionally substituted 2-bromobenzaldehyde (see Reaction scheme 5). Reaction with malonic acid in an appropriate solvent such as ethanol, in the presence of a base such as pyridine, at a suitable temperature, leads to the corresponding 2'-bromo-cinnamic acid. Said acid is activated with a reagent such as EDCI in the presence of a catalyst such as DMAP and a base such as NMM in DCM, and reacted with the capping group T to form the corresponding amide. Optionally substituted 2'-bromo-cinnamic acid amide can be reacted with 4-(4,4,5,5-tetramethyl-[1,3,2]dioxaborolan-2-yl)-3,6-dihydro-2H-pyridine-1-carboxylic acid tert-butyl ester in the presence of a base such as K₂CO₃ and a catalyst such as dichioro(1,1'-bis(diphenylphosphino)-ferrocene)palladium(II) DCM adduct, in an organic solvent such as DMF or toluene, at a suitable temperature to lead to the corresponding tetrahydropyridine. The resulting tetrahydropyridine and the cinnamic acid amide double bond can be hydrogenated in the presence of a catalyst, such as PtO₂ or Pd/C, to yield the protected piperidine. The side chain amide function can be reduced using a reagent such as LiAlH₄ or borane-THF complex in an appropriate inert solvent such as diethyl ether or THF at a suitable temperature to yield the Boc-protected A moiety.

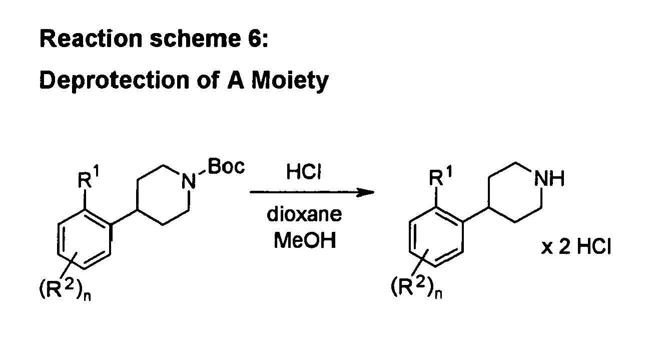

Generally, the starting material of Boc-protected phenylpiperidine (A moiety) can be deprotected in the presence of TFA/CH₂Cl₂, HCI/EtOAc, HCI/dioxane or HCl in MeOH/dioxane with or without a cation scavenger, such as dimethyl sulfide (DMS) before being subjected to the coupling procedure. It can be converted to the free base before being subjected to the coupling procedure or in some cases used as the salt.

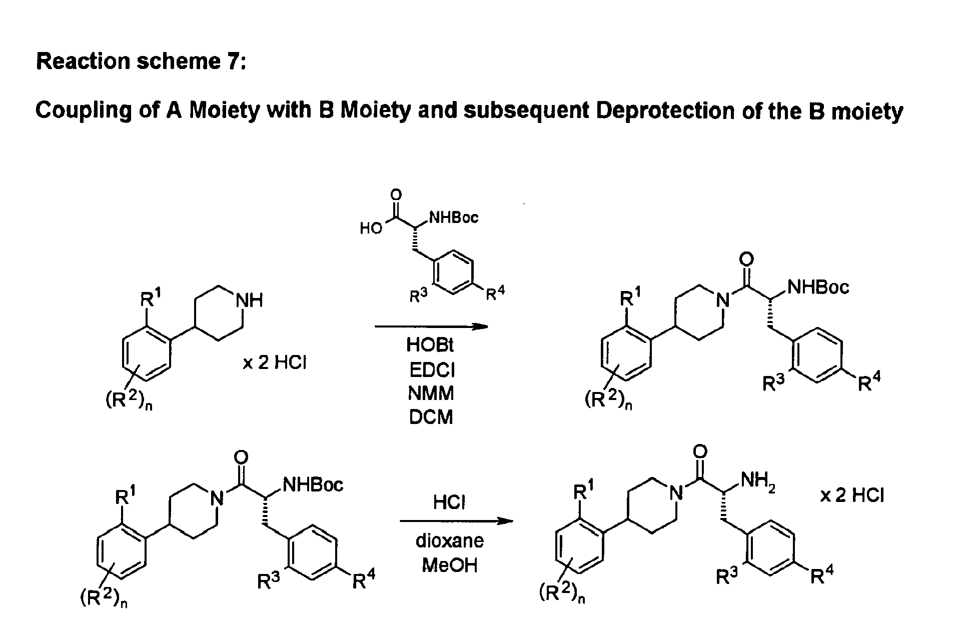

In coupling the A moiety with the protected B moiety shown in reaction scheme 7, an appropriate "A moiety" is coupled to a Boc-protected "B moiety" in the presence of EDCl/HOBt, a base such as N-methylmorpholine (NMM) and a solvent such as dichloromethane (DCM) followed by Boc deprotection with the aid of hydrochloric acid in a mixture of dioxane and methanol.

A suitable solvent, such as DCM, DMF, THF or a mixture of the above solvents, can be used for the coupling procedure. A suitable base includes triethylamine (TEA), diisopropyethylamine (DIEA), N-methylmorpholine (NMM), collidine or 2,6-lutidine.

A base may not be needed when EDCI/HOBt is used.

Generally after the reaction is completed, the reaction mixture can be diluted with an appropriate organic solvent, such as EtOAc, DCM or Et₂O, which is then washed with aqueous solutions, such as water, HCl, NaHSO₄, bicarbonate, NaH₂PO₄, phosphate buffer (pH 7), brine or any combination thereof. The reaction mixture can be concentrated and then be partitioned between an appropriate organic solvent and an aqueous solution. The reaction mixture can be concentrated and subjected to chromatography without aqueous workup.

### Analytical LC-MS

The compounds of the present invention according to formulas (I) and (II) were analyzed via analytical LC-MS. The conditions are summarized below.

### Analytical conditions summary:

LC10Advp-Pump (Shimadzu) with SPD-M10Avp UVNis diode array detector and QP2010 MS-detector in ESI+ modus with UV-detection at 214, 254 and 275 nm,
Column: Waters XTerra MS C18, 3.5 µm, 2.1 * 100 mm,
linear gradient with acetonitrile in water (0.1 % HCOOH)
Flow rate of 0,4 ml/min;

| | |
|---|---|
| Mobile Phase A: | water (0.1% HCOOH) |
| Mobile Phase B: | acetonitrile (0.1% HCOOH) |

### Gradient A:

**linear gradient from 1% to 95% acetonitrile in water (0.1% HCOOH)**

| | |
|---|---|
| 0.00 min | 1%B |
| 10.00 min | 95% B |
| 10.10 min | 99% B |
| 11.40 min | 99% B |
| 11.50 min | 1 % B |
| 13.00 min | Pump STOP |

### Gradient B:

**linear gradient from 1 % to 95% acetonitrile in water (0.1 % HCOOH)**

| | |
|---|---|
| 0.00 min | 1%B |
| 5.00 min | 95 % B |
| 5.10 min | 99 % B |
| 6.40 min | 99 % B |
| 6.50 min | 1 % B |
| 8.00 min | Pump STOP |

### Gradient C:

**linear gradient from 5% to 95% acetonitrile in water (0.1 % HCOOH)**

| | |
|---|---|
| 0.00 min | 5% B |
| 10.00 min | 95% B |
| 10.10 min | 99% B |
| 11.40 min | 99% B |
| 11.50 min | 1%B |
| 13.00 min | Pump STOP |

### Gradient D:

**linear gradient from 5% to 95% acetonitrile in water (0.1% HCOOH)**

| | |
|---|---|
| 0.00 min | 5% B |
| 5.00 min | 95 % B |
| 5.10 min | 99 % B |
| 6.40 min | 99 % B |
| 6.50 min | 1 % B |
| 8.00 min | Pump STOP |

The following describes the detailed examples of the invention which can be prepared via the reaction schemes 1 to 7.

**Table 1: Antagonists**

| | | | | | | |
|---|---|---|---|---|---|---|
| 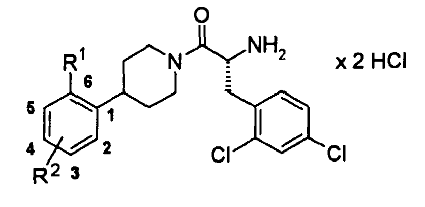 | | | | | | |

| | | | HPLC | | MS | |
|---|---|---|---|---|---|---|
| No. | R¹ | R² | t_{R} (min) | method | MW (calc.) | [M+H]⁺ (found) |
| 1 | 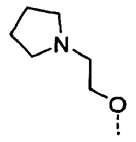 | H | 3.16 | A | 490.48 | 490 |
| 2 | 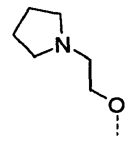 | 4-F | 2.93 | C | 508.47 | 508 |
| 3 | 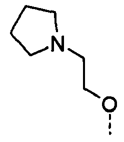 | 4-Cl | 3.20 | C | 524.93 | 524 |
| 4 | 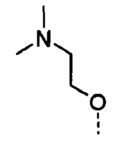 | 4-Cl | | | 498.89 | |
| 5 | 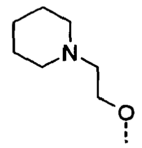 | 4-Cl | | | 538.95 | |
| 6 | 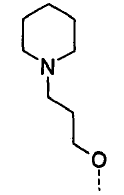 | 4-Cl | | | 552.97 | |
| 7 | 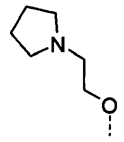 | 3-F 4-F | 3.99 | A | 526.46 | 526 |
| 8 | 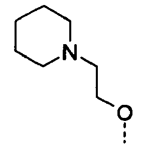 | 3-F 4-F | 4.03 | A | 540.49 | 540 |
| 9 | 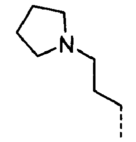 | 4-F | 3.98 | A | 508.47 | 506 |
| 10 | 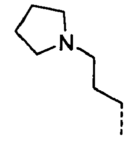 | 4-Cl | | | 524.93 | |
| 11 | 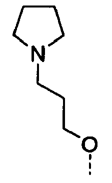 | H | 3.84 | A | 504.52 | 504 |

**Table 2: Agonists**

| | | | | | | |
|---|---|---|---|---|---|---|
| 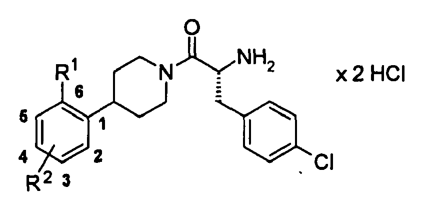 | | | | | | |

| | | | HPLC | | MS | |
|---|---|---|---|---|---|---|
| No. | R¹ | R² | t_{R} (min) | method | MW (calc.) | [M+H]⁺ (found) |
| 101 | 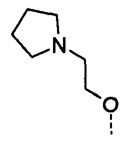 | H | 3.66 | A | 456.04 | 456 |
| 102 | 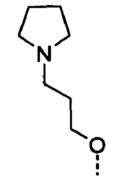 | H | 3.71 | A | 470.06 | 470 |
| 103 | 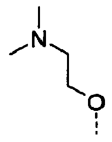 | H | 3.09 | C | 430.00 | 430 |
| 104 | 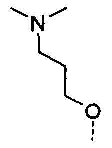 | H | 3.19 | C | 444.02 | 444 |
| 105 | 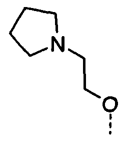 | 3-F | 3.47 | A | 474.03 | 474 |
| 106 | 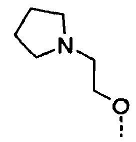 | 3-Cl | 3.70 | A | 490.48 | 490 |
| 107 | 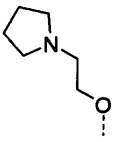 | 4-F | 3.54 | A | 474.03 | 474 |
| 108 | 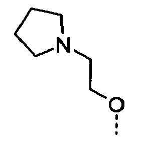 | 4-Cl | 3.76 | A | 490.48 | 490 |
| 109 | 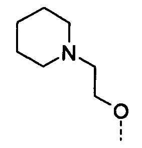 | 4-Cl | 3.80 | A | 504.51 | 508 |
| 110 | 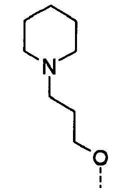 | 4-Cl | 3.67 | C | 518.53 | 518 |
| 111 | 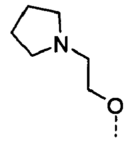 | 3-F 4-F | 3.84 | A | 492.02 | 492 |
| 112 | 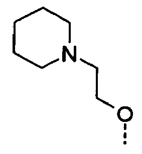 | 3-F 4-F | 3.95 | A | 506.04 | 506 |
| 113 | 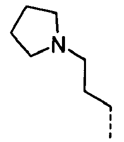 | H | 3.53 | B | 454.06 | 454 |
| 114 | 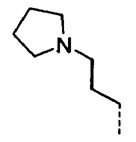 | 4-F | 3.96 | A | 472.05 | 472 |
| 115 | 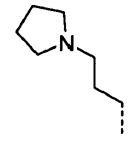 | 4-Cl | | | 488.51 | |

The following examples are provided to illustrate the invention and are not limiting the scope of the invention in any manner.

### Synthesis of Example 1:

### Intermediate 1a):

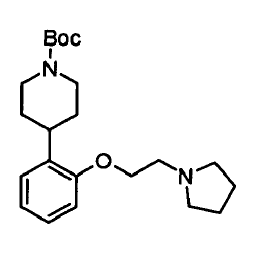

To a solution of 1-Boc-4-(2-hydroxy-phenyl)-piperidine (789 mg) in DMF (15 ml) was added 1-(2-chloroethyl)pyrrolidine hydrochloride (605 mg) and Cs₂CO₃ (3243 mg). The reaction was stirred at room temperature for 18 h. An additional amount of 1-(2-chloroethyl)pyrrolidine hydrochloride (483 mg) and Cs₂CO₃ (926 mg) were added and stirring at room temperature was continued for another 6 h. The reaction mixture was evaporated at 50 °C in vacuo to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated in vacuo to dryness. The residue was finally dried under high vacuum at room temperature overnight.

### Intermediate 1b):

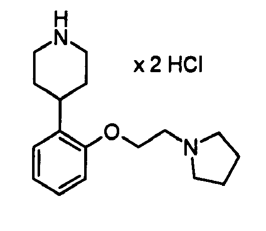

To Boc-protected intermediate 1a) (1002 mg) in methanol (5 ml) was added hydrogen chloride, 4.0 M sol. in 1,4-dioxane (25 ml) and the solution was stirred at room temperature for 2 h. The solvent was removed under reduced pressure. The residue was triturated in acetone (30 ml), filtered off, and washed with acetone (2 x 5 ml). Finally it was dried in vacuo at room temperature over P₂O₅ overnight to yield a white solid.

### Intermediate 1c):

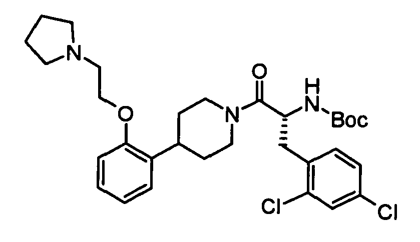

To Boc-D-2,4-dichlorophenylalanine (685 mg) in DCM (20 ml) was added the amine hydrochloride from 1b) (347 mg), N-methylmorpholine (311 µl), HOBt (230 mg) and the mixture was stirred for 30 min. EDCI (351 mg) was added and stirring was continued for 1 h. An additional amount of N-methylmorpholine (91 µl) was added and stirred overnight. The reaction mixture was evaporated in vacuo, diluted with EtOAc, washed with sat. Na₂CO₃, H₂O and brine. The aqueous layers were extracted with EtOAc. The combined organic layers were dried over Na₂SO₄, filtered and evaporated in vacuo to dryness.
Purification by flash chromatography yielded the title compound as white foam.

### Example 1:

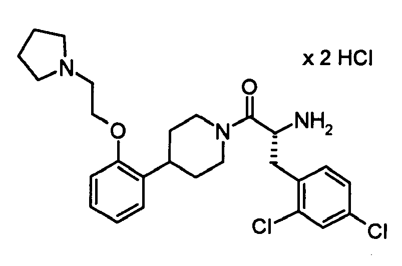

To Boc-protected intermediate 1c) (560 mg) in methanol (5 ml) was added hydrogen chloride, 4.0 M sol. in 1,4-dioxane (9 ml) and the solution was stirred for 90 min at room temperature. The reaction mixture was evaporated in vacuo to dryness. The residue was triturated in Et₂O, filtered off and washed with Et₂O to yield a beige solid.

### Synthesis of Example 6:

### Intermediate 6a):

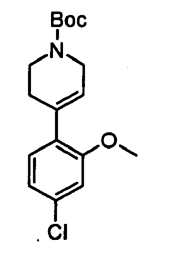

2-Bromo-5-chloroanisole (5.54 g), 1-(2(H)-pyridine-carboxylic acid-3,6-dihydro-4-(4,4,5,5-tetramethyl-1,3,2-dioxaborolan-2-yl)-tert.-butyl ester (7.73 g), dichloro(1,1'-bis(diphenylphosphino)-ferrocene)palladium(II) DCM adduct (1.22 g) and K₂CO₃ (10.36 g) were dissolved in degassed DMF in a dry apparatus under argon and the mixture was degassed again by evacuation followed by refilling with argon. The resulting suspension was heated in an oil bath at 85°C overnight. The mixture was cooled, filtered through Celite and evaporated to dryness. The crude product was purified by flash chromatography to yield a clear yellowish oil.

### Intermediate 6b):

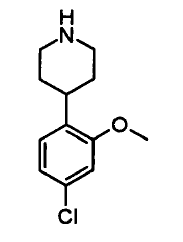

Intermediate 6a) (2.18 g) was dissolved in EtOH (80 ml) and AcOH (80 ml) under argon. Platinum(IV) oxide (0.23 g) was added and the reaction mixture was placed under an H₂ atmophere using a balloon. The reaction mixture was then stirred at room temperature for 120 min. The reaction mixture was filtered through Celite and evaporated to dryness in vacuo. The residue was coevaporated with toluene (3 x 40 ml). The crude product was purified by flash chromatography to yield a clear colorless oil.

### Intermediate 6c):

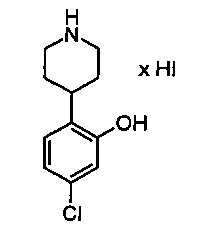

To a solution of intermediate 6b) (1.56 g) in AcOH (6.5 ml) was added hydroiodic acid (5.2 ml of a 57 wt.% aq. solution) and the mixture was heated under reflux (oil bath at 140°C) in an argon atmosphere for 2 h. The reaction mixture was cooled to room temperature and then evaporated to dryness in vacuo. The residue was coevaporated with toluene (3 x 30 ml). The crude product was triturated in Et₂O (40 ml), the insoluble compound was filtered off and washed with Et₂O (10 ml). Finally the product was dried in vacuo over P₂O₅ at room temperature overnight to yield a white solid.

### Intermediate 6d):

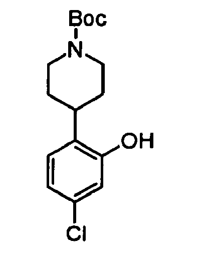

To a solution of intermediate 6c) (1.55 g) in DMF (10 ml) was added DIEA (0.88 ml) followed by di-tert.-butyl-dicarbonate (1.01 g). The reaction mixture was stirred at room temperature for 4 h. The mixture was evaporated in vacuo to dryness and partitioned between 0.5 M HCl (50 ml) and EtOAc (100 ml). The organic layer was washed with water (25 ml) and brine (30 ml). The organic layer was dried with MgSO₄ and evaporated in vacuo to dryness to yield a yellowish solid. The solid residue was triturated in EtOAc (1 ml) and Et₂O (10 ml), and left in the fridge overnight to complete crystallization of the product. The precipitate was then filtered off, washed with cold Et₂O (1 ml), and finally dried in vacuo at room temperature over P₂O₅ overnight. The product was obtained in form of a white solid.

### Intermediate 6e):

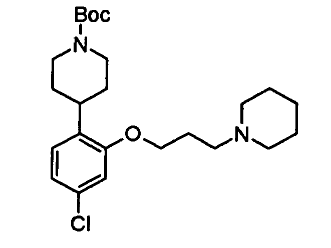

To a solution of intermediate 6d) (468 mg) in DMF (8 ml) was added 1-(3-chloropropyl)piperidine hydrochloride (373 mg) and Cs₂CO₃ (1710 mg). The reaction was stirred at room temperature for 18 h. An additional amount of 1-(3-chloropropyl)piperidine hydrochloride (297 mg) and Cs₂CO₃ (489 mg) were added and stirring at room temperature was continued for 3 d. The reaction mixture was evaporated at 40 °C in vacuo to dryness and the residue was partitioned between EtOAc and water. The aqueous layer was extracted with EtOAc. The combined organic layer was washed with water and brine. The organic layer was dried over Na₂SO₄ and evaporated in vacuo to dryness. The crude product was purified using flash chromatography.

### Intermediate 6f):

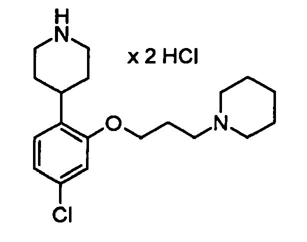

To Boc-protected intermediate 6e) (651 mg) in methanol (3 ml) was added hydrogen chloride, 4.0 M sol. in 1,4-dioxane (11 ml) and the solution was stirred at room temperature for 90 min. The solvent was removed under reduced pressure. The residue was triturated in acetone and Et₂O, filtered off, and washed with Et₂O. Finally it was dried in vacuo at room temperature over P₂O₅ overnight to yield a white solid.

### Intermediate 6g):

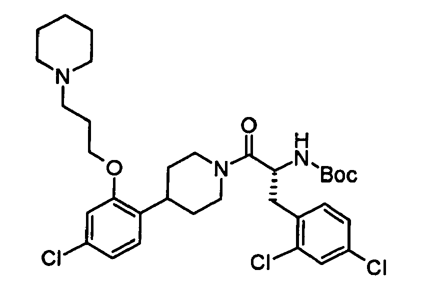

To Boc-D-2,4-dichlorophenylalanine (33 mg) in DCM (2 ml) was added amine hydrochloride from 6f) (30 mg), N-methylmorpholine (23 µl), HOBt (17 mg) and the mixture was stirred for 30 min. EDCI (26 mg) was added and stirring was continued for 1 h. An additional amount of N-methylmorpholine (7 µl) was added and stirred overnight. The reaction mixture was evaporated in vacuo, diluted with EtOAc, washed with sat. Na₂CO₃, H₂O and brine. The aqueous layers were extracted with EtOAc. The combined organic layers were dried over Na₂SO₄, filtered and evaporated in vacuo to dryness.
Purification by flash chromatography yielded the title compound as white foam.

### Example 6:

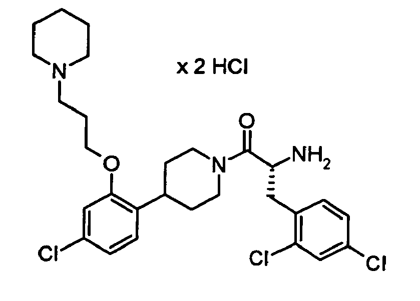

To Boc-protected intermediate 6g) (40 mg) in methanol (0.3 ml) was added hydrogen chloride, 4.0 M sol. in 1,4-dioxane (0.6 ml) and the solution was stirred at room temperature for 2 h. The reaction mixture was evaporated in vacuo to dryness. The residue was triturated in acetone and Et₂O, filtered off and washed with Et₂O to yield a white solid.

### Synthesis of Example 8:

### Intermediate 8a):

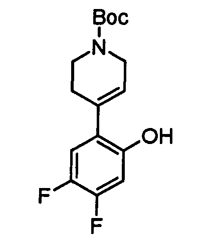

2-Bromo-4,5-difluorophenol (2857 µl), N-Boc-1,2,3,6-tetrahydropyridine-4-boronic acid pinacol ester (7.73 g), potassium carbonate (10.36 g) and dichloro(1,1'-bis(diphenylphosphino)-ferrocene)palladium(II) DCM adduct (1.22 g) were dissolved in DMF (150 ml) in a dry apparatus under argon and the mixture was degassed by bubbling with argon for 30 min. The orange suspension was then heated under argon in an oil bath at 85°C for 1 d to give a dark purple suspension. The reaction mixture was filtered through Celite and evaporated to dryness in vacuo. The crude product was purified by flash chromatography to yield pale green crystals.

### Intermediate 8b):

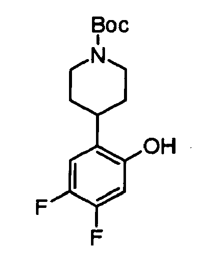

Intermediate 8a) (1404 mg) was dissolved in EtOH (50 ml) and AcOH (50 ml) and platinum(IV) oxide (102 mg) was added. The reaction mixture was evacuated three times and purged with hydrogen. The reaction mixture was then stirred at room temperature for 2 h. The reaction mixture was filtered and evaporated to dryness in vacuo. The residue was coevaporated with toluene (3 x 75 ml) and was finally dried under high vacuum at room temperature overnight to yield a beige solid.

### Intermediate 8c):

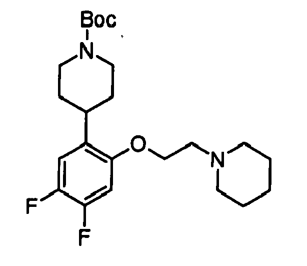

To a solution of intermediate 8b) (674 mg) in DMF (15 ml) was added 1-(2-chloroethyl)piperidine hydrochloride (605 mg) and Cs₂CO₃ (2453 mg). The reaction was stirred at room temperature for 2 d. An additional amount of 1-(2-chloroethyl)piperidine hydrochloride (199 mg) and Cs₂CO₃ (352 mg) was added and stirring at room temperature was continued for another 3 d. The reaction mixture was evaporated at 50°C in vacuo to dryness and the residue was partitioned between Et₂O (75 ml) and water (25 ml). The aqueous layer was extracted with Et₂O (25 ml). The combined organic layer was washed with water (10 ml) and brine (15 ml). The organic layer was dried over Na₂SO₄ and evaporated in vacuo to dryness. The residue was finally dried under high vacuum at room temperature overnight. The crude product was purified by flash chromatography.

### Intermediate 8d):

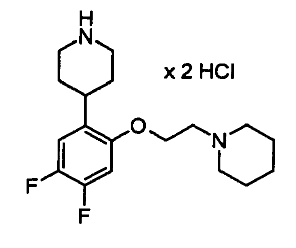

To Boc-protected intermediate 8c) (490 mg) in methanol (2 ml) and dioxane (10 ml) was added hydrogen chloride, 4.0 M sol. in 1,4-dioxane (10 ml) and the solution was stirred at room temperature for 30 min. The solvent was removed under reduced pressure. The residue was triturated in acetone and Et₂O, filtered off, and washed with Et₂O. Finally it was dried in vacuo at room temperature over P₂O₅ overnight to yield an off-white solid.

### Intermediate 8e):

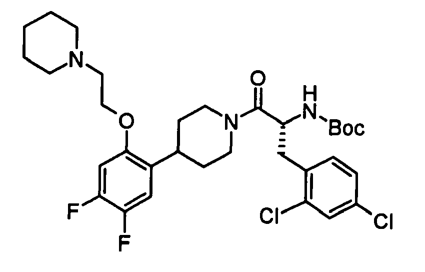

To Boc-D-2,4-dichlorophenylalanine (90 mg) in DCM (2 ml) was added amine hydrochloride from 8d) (99 mg), N-methylmorpholine (41 µl), HOBt (42 mg) and the mixture was stirred for 30 min. EDCI (72 mg) was added and stirring was continued for 1 h. An additional amount of N-methylmorpholine (20 µl) was added and stirred overnight. The reaction mixture was diluted with EtOAc, washed with sat. Na₂CO₃ and brine. The organic layer was dried over Na₂SO₄, filtered and evaporated in vacuo to dryness.

Purification by flash chromatography yielded the title compound as colorless glassy solid.

### Example 8:

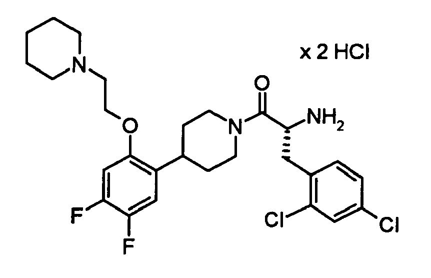

To Boc-protected intermediate 8e) (128 mg) in methanol (1 ml) and dioxane (5 ml) was added hydrogen chloride, 4.0 M sol. in 1,4-dioxane (5 ml) and the solution was stirred for 30 min at room temperature. The reaction mixture was evaporated in vacuo to dryness. The residue was triturated in Et₂O, filtered off and washed with Et₂O to yield an off-white solid.

### Synthesis of Example 9:

### Intermediate 9a):

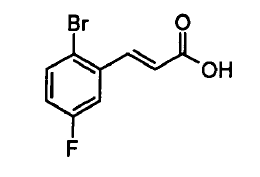

A mixture of 2-bromo-5-fluorobenzaldehyde (10.15 g), malonic acid (5.72 g) and pyridine (1.5 ml) in ethanol (25 ml) was kept under reflux for 7.5 h. After cooling in an ice bath the crystal mass was filtered off. The crystals were washed with cold ethanol (10 ml) and then twice with diethyl ether (10 ml each). The residue was suspended in ethanol (60 ml) and kept under reflux for 2-3 h. The mixture was cooled and filtered and the solid was dried under reduced pressure. The product was obtained in form of colorless needles.

### Intermediate 9b):

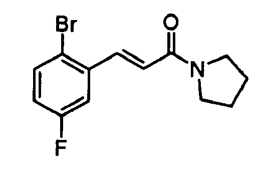

Intermediate 9a) (2451 mg), pyrrolidine (918 µl), EDCI (2109 mg) and DMAP (100 mg) were dissolved in DCM (100 ml) and stirred overnight. The reaction mixture was poured into water (100 ml) and the organic layer was separated. The aqueous layer was extracted twice with DCM. The combined organics were washed three times with with 0.5 N HCl (30 ml each), three times with 1 M sodium hydroxide solution and brine, dried over Na₂SO₄ and the solvent was removed under reduced pressure. The crude product was purified by flash chromatography to yield a white solid.

### Intermediate 9c):

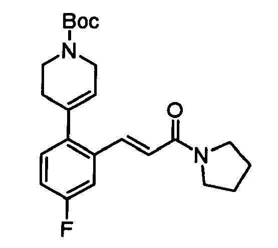

Intermediate 9b) (1130 mg), N-Boc-1,2,3,6-tetrahydropyridine-4-boronic acid pinacol ester (1231 mg), potassium carbonate (1571 mg) and dichloro(1,1'-bis(diphenyl-phosphino)-ferrocene)palladium(II) DCM adduct (188 mg) were dissolved in DMF (50 ml) in a dry apparatus under argon and the mixture was degassed by bubbling with argon for 30 min. The orange suspension was then heated under argon in an oil bath at 85°C for 1 d. The reaction mixture was filtered through Celite and evaporated to dryness in vacuo. The crude product was purified by flash chromatography to yield a beige solid.

### Intermediate 9d):

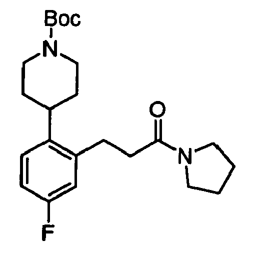

Intermediate 9c) (1459 mg) was dissolved in ethanol (50 ml) and 10% palladium on activated carbon (150 mg) was added. The reaction mixture was purged three times with hydrogen (5 bar) and stirred under hydrogen atmosphere (10 bar) for 3 d. The crude mixture was filtered through Celite and the solvent was removed under reduced pressure to yield a yellow-grey oil.

### Intermediate 9e):

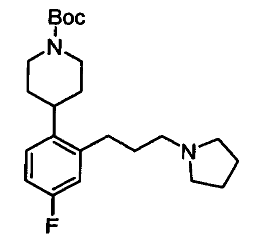

Intermediate 9d) (1472 mg) in diethyl ether (20 ml) was slowly added to a mixture of lithium aluminum hydride (207 mg) and diethyl ether (30 ml) at 0°C. After addition the reaction mixture was stirred at 0°C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a pale yellow oil.

### Intermediate 9f):

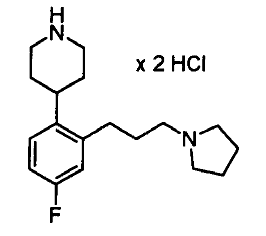

To intermediate 9e) (654 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml) and the product was filtered off to yield an off-white solid.

### Intermediate 9g):

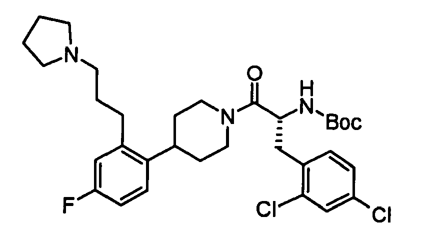

To Boc-D-2,4-dichlorophenylalanine (90 mg) in DCM (2 ml) was added amine hydrochloride from 9f) (91 mg), N-methylmorpholine (41 µl), HOBt (42 mg) and the mixture was stirred for 30 min. EDCI (72 mg) was added and stirring was continued for 1 h. An additional amount of N-methylmorpholine (20 µl) was added and stirred overnight. The reaction mixture was diluted with EtOAc, washed with sat. Na₂CO₃ and brine. The organic layers was dried over Na₂SO₄, filtered and evaporated in vacuo to dryness.
Purification by flash chromatography yielded the title compound as colorless glassy solid.

### Example 9:

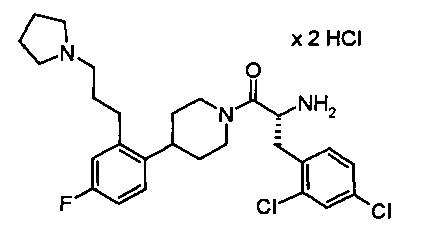

To Boc-protected intermediate 9g) (83 mg) in methanol (1 ml) and dioxane (5 ml) was added hydrogen chloride, 4.0 M sol. in 1,4-dioxane (5 ml) and the solution was stirred for 30 min at room temperature. The reaction mixture was evaporated in vacuo to dryness. The residue was triturated in Et₂O, filtered off and washed with Et₂O to yield an off-white solid.

### Synthesis of Example 10:

### Intermediate 10a):

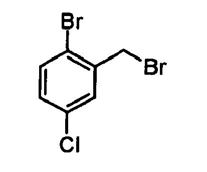

2-Bromo-5-chlorotoluene (8.26 ml) and N-bromosuccinimide (11.03 g) in carbontetrachloride (50 ml) were treated with a catalytic amount of benzoylperoxide (100 mg) and heated under reflux until the reaction had reached completion as monitored by TLC. The reaction mixture was then allowed to cool and filtered. The filtrate was washed twice with water and brine, dried over sodium sulfate and concentrated in vacuo.

### Intermediate 10b):

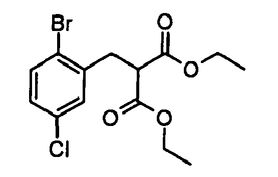

To a solution of sodium ethoxide (2.79 g) in ethanol (30 ml) was added diethyl malonate (6.54 ml) and the mixture was stirred for 1 h at room temperature. The mixture was cooled in an ice-bath and intermediate 10a) (11.67 g) was slowly added and the reaction mixture was kept under reflux overnight. The reaction mixture was evaporated in vacuo and the residue was portioned between diethyl ether and water and the aqueous layer extracted two times with diethyl ether. The combined organic layer was washed twice with water and brine. The combined organic layer was dried over Na₂SO₄ and evaporated in vacuo to dryness. The product was purified by Kugelrohr distillation. The fractions which distilled off between 160 and 230°C at 0.2-0.3 mbar were collected.

### Intermediate 10c):

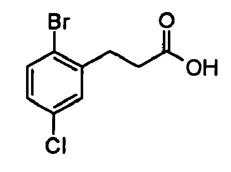

Intermediate 10b) (8.98 g) was heated under reflux in 1.8 M KOH in H₂O/EtOH (60 ml) for 5 h. After evaporation of the ethanol an additional amount of KOH (18 g) was added to the residue, and the reaction mixture was stirred for 2 h at 100 °C. The reaction mixture was diluted with 100 ml of H₂O, extracted with Et₂O and the organic layer was washed with H₂O. The combined aqueous layer was cooled with ice/H₂O and acidified with 50 % H₂SO₄ to pH 1. The precipitate was extracted twice with Et₂O (100 ml each) and the organic layers were washed with water and brine. The combined organic layers were dried over Na₂SO₄ and evaporated in vacuo to dryness. The residue was triturated in hexane and less Et₂O, then filtered and washed with hexane and less Et₂O. The solid residue was decarboxylated by heating at 200 °C. The development of CO₂ ceased after 20 min and the melt was cooled to room temperature. The residue was crushed with a glass rod to get a homogeneous beige solid.

### Intermediate 10d):

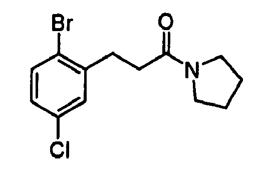

Intermediate 10c) (2320 mg), pyrrolidine (808 µl), EDCI (1856 mg) and DMAP (100 mg) were dissolved in DCM (100 ml) and stirred overnight. The reaction mixture was poured into water (100 ml) and the organic layer was separated. The aqueous layer was extracted twice with DCM. The combined organics were washed three times with with 0.5 N HCl (30 ml each), three times with 1 M sodium hydroxide solution and brine, dried over Na₂SO₄ and the solvent was removed under reduced pressure. The product was obtained as off-white solid after purification by flash chromatography.

### Intermediate 10e):

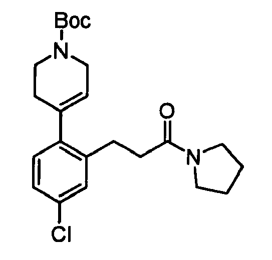

Intermediate 10d) (1901 mg), N-Boc-1,2,3,6-tetrahydropyridine-4-boronic acid pinacol ester (1948 mg), potassium carbonate (3317 mg) and dichloro(1,1'-bis(diphenylphosphino)-ferrocene)palladium(II) DCM adduct (294 mg) were dissolved in DMF (70 ml) in a dry apparatus under argon and the mixture was degassed by bubbling with argon for 30 min. The orange suspension was then heated under argon in an oil bath at 85°C for 3 days to give a dark purple suspension. The reaction mixture was filtered through Celite and evaporated to dryness in vacuo. The crude product was purified by column chromatography.

### Intermediate 10f):

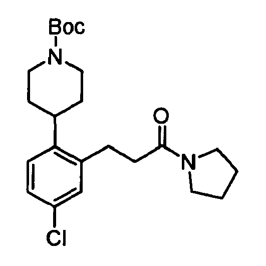

Intermediate 10e) (2372 mg) was dissolved in EtOH (50 ml) and AcOH (50 ml) and platinum(IV) oxide (129 mg) was added. The reaction mixture was evacuated three times and purged with hydrogen. The reaction mixture was then stirred at room temperature for 2 h. The reaction mixture was filtered and evaporated to dryness in vacuo. The residue was coevaporated with toluene (3 x 75 ml) and was finally dried under high vacuum at RT overnight.

### Intermediate 10g):

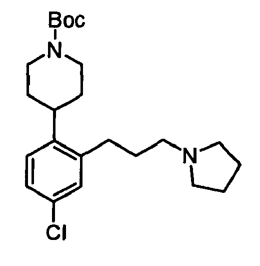

Intermediate 10f) (1687 mg) in diethyl ether (20 ml) was slowly added to a mixture of lithium aluminum hydride (228 mg) and diethyl ether (30 ml) at 0°C. After addition, the reaction mixture was stirred at 0°C for 1 h. The reaction mixture was hydrolyzed with a minimum amount of water. The inorganic precipitate was filtered off and washed twice with diethyl ether. The combined filtrates were dried over sodium sulfate, filtered, and the solvent was removed under reduced pressure. The crude product was purified by flash chromatography to yield a colorless oil.

### Intermediate 10h):

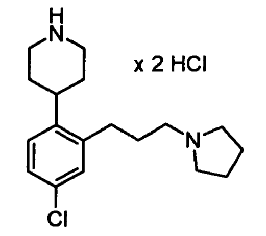

To intermediate 10g) (864 mg) in dioxane (10 ml) and methanol (2 ml) was added hydrogen chloride, 4.0 M sol. in 1,4-dioxane (10 ml) and the solution was stirred for 30 min at room temperature. The solvent was removed under reduced pressure, the residue was triturated with acetone (5 ml) and diethyl ether (50 ml) and the product was filtered off. The product was obtained as off-white solid.

### Intermediate 10i):

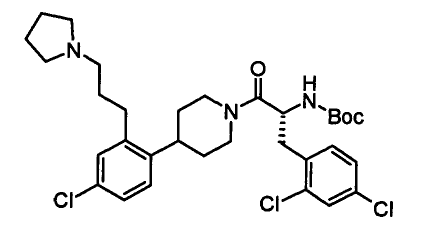

To Boc-D-2,4-dichlorophenylalanine (90 mg) in DCM (2 ml) was added amine hydrochloride from 10h) (95 mg), N-methylmorpholine (41 µl), HOBt (42 mg) and the mixture was stirred for 30 min. EDCI (72 mg) was added and stirring was continued for 1 h. An additional amount of N-methylmorpholine (20 µl) was added and stirred overnight. The reaction mixture was diluted with EtOAc, washed with sat. Na₂CO₃ and brine. The organic layers was dried over Na₂SO₄, filtered and evaporated in vacuo to dryness. Purification by flash chromatography yielded the title compound as colorless glassy solid.

### Example 10:

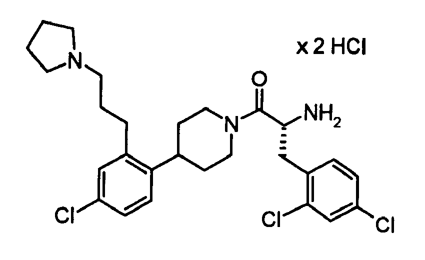

To Boc-protected intermediate 11i) (84 mg) in methanol (1 ml) and dioxane (5 ml) was added hydrogen chloride, 4.0 M sol. in 1,4-dioxane (5 ml) and the solution was stirred for 30 min at room temperature. The reaction mixture was evaporated in vacuo to dryness. The residue was triturated in Et₂O, filtered off and washed with Et₂O to yield an off-white solid.

### Synthesis of Example 101:

### Intermediate 101a):

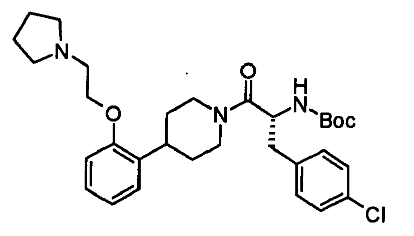

To Boc-D-4-chlorophenylalanine (574 mg) in DCM (30 ml) was added amine hydrochloride from 1b) (500 mg), N-methylmorpholine (448 µl), HOBt (330 mg) and the mixture was stirred for 30 min. EDCI (505 mg) was added and stirring was continued for 1 h. An additional amount of N-methylmorpholine (131 µl) was added and stirred overnight. The reaction mixture was evaporated in vacuo, diluted with EtOAc, washed with sat. Na₂CO₃, H₂O and brine. The aqueous layers were extracted with EtOAc. The combined organic layers were dried over Na₂SO₄, filtered and evaporated in vacuo to dryness. Purification by flash chromatography yielded the title compound as white foam.

### Example 101:

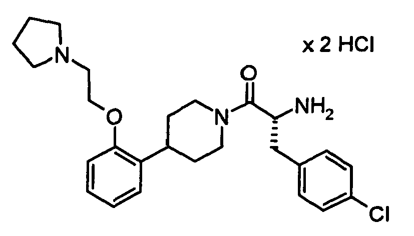

To Boc-protected intermediate 101 a) (721 mg) in methanol (4 ml) was added hydrogen chloride, 4.0 M sol. in 1,4-dioxane (10 ml) and the solution was stirred for 90 min at room temperature. The reaction mixture was evaporated in vacuo to dryness. The residue was triturated in Et₂O, filtered off and washed with Et₂O to yield a beige solid.

### Synthesis of Example 110:

### Intermediate 110a):

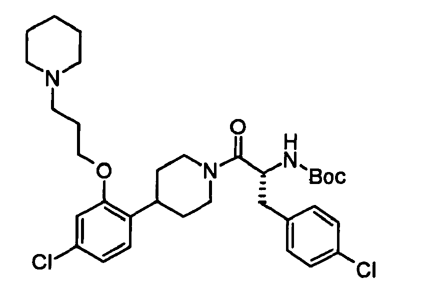

To Boc-D-4-chlorophenylalanine (29 mg) in DCM (2 ml) was added amine hydrochloride from 6f) (30 mg), N-methylmorpholine (23 µl), HOBt (17 mg) and the mixture was stirred for 30 min. EDCI (26 mg) was added and stirring was continued for 1 h. An additional amount of N-methylmorpholine (7 µl) was added and stirred overnight. The reaction mixture was evaporated in vacuo, diluted with EtOAc, washed with sat. Na₂CO₃, H₂O and brine. The aqueous layers were extracted with EtOAc. The combined organic layers were dried over Na₂SO₄, filtered and evaporated in vacuo to dryness. Purification by flash chromatography yielded the title compound as white foam.

### Example 110:

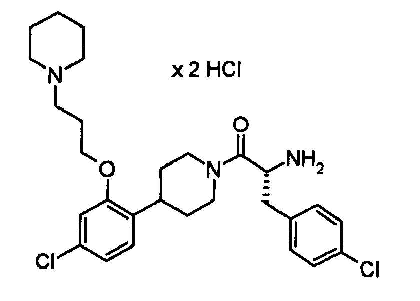

To Boc-protected intermediate 110a) (39 mg) in methanol (0.3 ml) was added hydrogen chloride, 4.0 M sol. in 1,4-dioxane (0.6 ml) and the solution was stirred at room temperature for 2 h. The reaction mixture was evaporated in vacuo to dryness. The residue was triturated in acetone and Et₂O, filtered off and washed with Et₂O to yield a white solid.

### Synthesis of Example 112:

### Intermediate 112a):

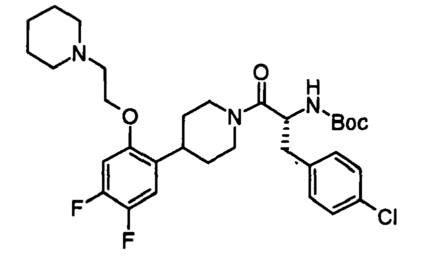

To Boc-D-4-chlorophenylalanine (80 mg) in DCM (2 ml) was added amine hydrochloride from 8d) (99 mg), N-methylmorpholine (41 µl), HOBt (42 mg) and the mixture was stirred for 30 min. EDCI (72 mg) was added and stirring was continued for 1 h. An additional amount of N-methylmorpholine (20 µl) was added and stirred overnight. The reaction mixture was diluted with EtOAc, washed with sat. Na₂CO₃ and brine. The organic layer was dried over Na₂SO₄, filtered and evaporated in vacuo to dryness.
Purification by flash chromatography yielded the title compound as colorless glassy solid.

### Example 112:

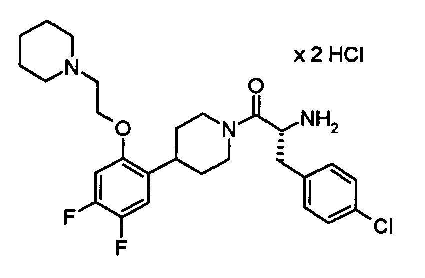

To Boc-protected intermediate 112a) (119 mg) in methanol (1 ml) and dioxane (5 ml) was added hydrogen chloride, 4.0 M sol. in 1,4-dioxane (5 ml) and the solution was stirred for 30 min at room temperature. The reaction mixture was evaporated in vacuo to dryness. The residue was triturated in Et₂O, filtered off and washed with Et₂O to yield an off-white solid.

### Synthesis of Example 114:

### Intermediate 114a):

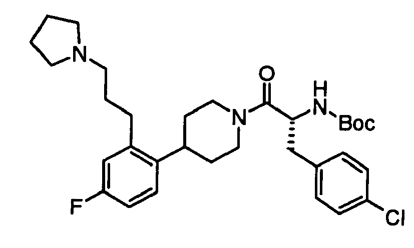

To Boc-D-4-chlorophenylalanine (80 mg) in DCM (2 ml) was added amine hydrochloride from 9f) (91 mg), N-methylmorpholine (41 µl), HOBt (42 mg) and the mixture was stirred for 30 min. EDCI (72 mg) was added and stirring was continued for 1 h. An additional amount of N-methylmorpholine (20 µl) was added and stirred overnight. The reaction mixture was diluted with EtOAc, washed with sat. Na₂CO₃ and brine. The organic layer was dried over Na₂SO₄, filtered and evaporated in vacuo to dryness.
Purification by flash chromatography yielded the title compound as colorless glassy solid.

### Example 114:

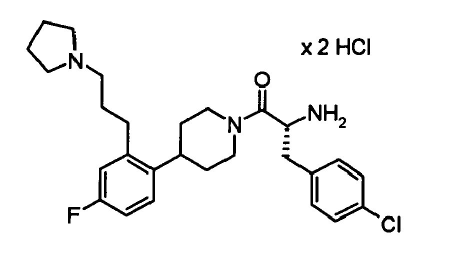

To Boc-protected intermediate 114a) (81 mg) in methanol (1 ml) and dioxane (5 ml) was added hydrogen chloride, 4.0 M sol. in 1,4-dioxane (5 ml) and the solution was stirred for 30 min at room temperature. The reaction mixture was evaporated in vacuo to dryness. The residue was triturated in Et₂O, filtered off and washed with Et₂O to yield an off-white solid.

### Synthesis of Example 115:

### Intermediate 115a):

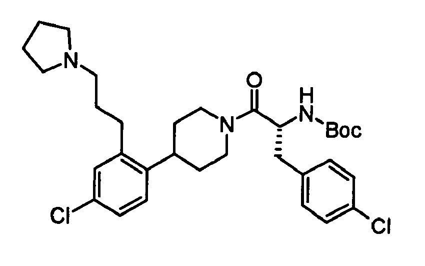

To Boc-D-4-chlorophenylalanine (80 mg) in DCM (2 ml) was added amine hydrochloride from 10h) (95 mg), N-methylmorpholine (41 µl), HOBt (42 mg) and the mixture was stirred for 30 min. EDCI (72 mg) was added and stirring was continued for 1 h. An additional amount of N-methylmorpholine (20 µl) was added and stirred overnight. The reaction mixture was diluted with EtOAc, washed with sat. Na₂CO₃ and brine. The organic layer was dried over Na₂SO₄, filtered and evaporated in vacuo to dryness.
Purification by flash chromatography yielded the title compound as colorless glassy solid.

### Example 115:

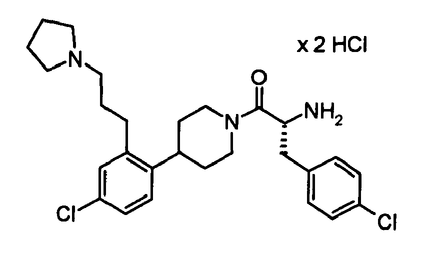

To Boc-protected intermediate 115a) (83 mg) in methanol (1 ml) and dioxane (5 ml) was added hydrogen chloride, 4.0 M sol. in 1,4-dioxane (5 ml) and the solution was stirred for 30 min at room temperature. The reaction mixture was evaporated in vacuo to dryness. The residue was triturated in Et₂O, filtered off and washed with Et₂O to yield an off-white solid.

### BIOLOGICAL ASSAYS

### A. Binding Assay

A membrane binding assay is used to identify competitive inhibitors of fluorescence labeled NDP-alpha-MSH binding to HEK293 cell membrane preparations expressing human melanocortin receptors.

The test compound or unlabeled NDP-alpha-MSH is dispensed at varying concentrations to a 384 well microtiter plate. Fluorescence labeled NDP-alpha-MSH is dispensed at a single concentration, followed by addition of membrane preparations. The plate is incubated for 5 h at room temperature.

The degree of fluorescence polarization is determined with a fluorescence polarization microplate reader.

### B. Functional Assay

Agonistic activity of human melanocortin receptors is determined in a homogeneous membrane based assay. Competition between unlabeled cAMP and a fixed quantity of fluorescence labeled cAMP for a limited number of binding sites on a cAMP specific antibody is revealed by fluorescence polarization.

The test compound or unlabeled NDP-alpha-MSH is dispensed at varying concentrations to a 384 well microtiter plate. Membrane preparations from HEK293 cells expressing the human melanocortin receptors are added. After a short preincubation period, an appropriate amount of ATP, GTP and the cAMP antibody is added and the plate is further incubated before the fluorescence labeled cAMP conjugate is dispensed. The plate is incubated for 2 h at 4°C before it is read on a fluorescence polarization microplate reader. The amount of cAMP produced as a response to a test compound is compared to the production of cAMP resulting from stimulation with NDP-alpha-MSH.

Representative compounds of the present invention were tested and found to bind to the melanocortin-4 receptor. These compounds were generally found to have IC₅₀ values less than 2 µM. Representative compounds of the present invention were also tested in the functional assay and found generally to activate the melanocortin-4 receptor with EC₅₀ values less than 1 µM.

**Table 3: Biological data for selected examples of the invention**

| Example | hMC-4R binding assay IC₅₀/µM | hMC-4R functional assay EC₅₀/µM | % activation functional assay |
|---|---|---|---|
| SHU9119 | 0.0019 | | 7 |
| NDP-α-MSH | 0.0011 | 0.0034 | 100 |
| 1 | 0.13 | | no activation |
| 2 | 0.064 | | 22 |
| 3 | 0.028 | | no activation |
| 7 | 0.057 | | 29 |
| 101 | 0.47 | 1.6 | 78 |
| 105 | 0.26 | 0.57 | 90 |
| 106 | 0.15 | 0.23 | 87 |
| 110 | 0.19 | 0.28 | 73 |
| 111 | 0.31 | 0.17 | 75 |
| 112 | 0.26 | 0.11 | 73 |
| 113 | 0.79 | 1.1 | 78 |
| 114 | 0.29 | 0.64 | 89 |

### C. In Vivo Food Intake Models

### 1. Spontaneous Feeding Paradigm

Food intake in rats is measured after i.p. or p.o. administration of the test compound (see e.g. Chen, A.S. et al. Transgenic Res 2000 Apr;9(2):145-54).

### 2. Model of LPS and Tumor-Induced Cachexia

Prevention or amelioration of anorexia induced by lipopolysaccharide (LPS) administration or cachexia induced by tumor growth is determined upon i.p. or p.o. administration of test compounds to rats (see e.g. Marks, D.L.; Ling, N and Cone, R.D. Cancer Res 2001 Feb 15;61 (4):1432-8).

### D. Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water ad lib and are kept on a normal light/dark cycle. Studies are conducted during the light cycle.

### 1. Conditioning to Supine Restraint for Ex Copula Reflex Tests

This conditioning takes about 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation, animals are given a treat to ensure positive reinforcement.

### 2. Ex Copula Reflex Tests

Rats are gently restrained in a supine position with their anterior torso placed inside a cylinder of adequate size to allow for normal head and paw grooming. For a 400 - 500 gram rat, the diameter of the cylinder is approximately 8 cm. The lower torso and hind limbs are restrained with a nonadhesive material (vetrap). An additional piece of vetrap with a hole in it, through which the glans penis will be passed, is fastened over the animal to maintain the preputial sheath in a retracted position. Penile responses will be observed, typically termed ex copula genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how, and if, an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### E. Models of Female Sexual Dysfunction

Rodent assays relevant to female sexual receptivity include the behavioral model of lordosis and direct observations of copulatory activity. There is also a urethrogenital reflex model in anesthetized spinally transected rats for measuring orgasm in both male and female rats. These and other established animal models of female sexual dysfunction are described in McKenna KE et al, A Model For The Study of Sexual Function In Anesthetized Male And Female Rats, Am. J. Physiol. (Regulatory Integrative Comp. Physiol 30): R1276-R1285, 1991; McKenna KE et al, Modulation By Peripheral Serotonin of The Threshold For Sexual Reflexes In Female Rats, Pharm. Bioch. Behav., 40:151-156, 1991; and Takahashi LK et al, Dual Estradiol Action In The Diencephalon And The Regulation of Sociosexual Behavior In Female Golden Hamsters, Brain Res., 359:194-207, 1985.

### Examples of a Pharmaceutical Composition

As a specific embodiment of an oral composition of a compound of the present invention, 25 mg of Example 1 is formulated with sufficient finely divided lactose to provide a total amount of 580 to 590 mg to fill a size 0 hard gelatin capsule.

As another specific embodiment of an oral composition of a compound of the present invention, 35 mg of Example 102 is formulated with sufficient finely divided lactose to provide a total amount of 580 to 590 mg to fill a size 0 hard gelatin capsule.

While the invention has been described and illustrated in reference to certain preferred embodiments thereof, those skilled in the art will appreciate that various changes, modifications and substitutions can be made therein without departing from the spirit and scope of the invention. For example, effective dosages, other than the preferred doses as set forth above, may be applicable as a consequence of the specific pharmacological responses observed and may vary depending upon the particular active compound selected, as well as from the type of formulation and mode of administration employed, and such expected variations or differences in the results are contemplated in accordance with the objects and practices of the present invention. It is intended, therefore, that the invention be limited only by the scope of the claims which follow and that such claims be interpreted as broadly as is reasonable.

## Claims

1. A compound according to formula (I) 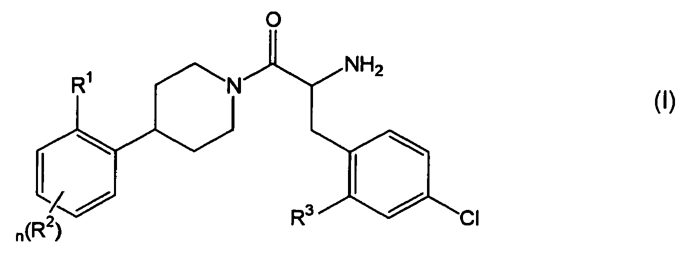 and enantiomers, diastereomers, tautomers, solvates and pharmaceutically acceptable salts thereof,
wherein
R¹ is -(CH₂)₁-T,
-O-(CH₂)ₘ-T,
T is NR⁵R⁶,
morpholine, 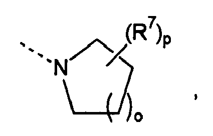 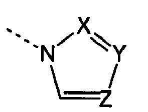
R⁵ and R⁶ are independently
C₁₋₆-alkyl,
C₂₋₆-alkenyl,
C₂₋₆-alkinyl,
C₂₋₆-alkylene-O-C₁₋₆-alkyl,
R⁷ is halogen,
CN,
OH,
C₁₋₆-alkyl, optionally substituted with 1 to 3 substituents selected from halogen,
CN and OH,
O-C₁₋₆-alkyl optionally substituted with 1 to 3 substituents selected from halogen, CN and OH,
X is CH, N,
Y is CH, N,
Z is CH, N,
R² is F,
Cl,
methyl,
R³ is Cl,
methyl,
I is 3, 4,
m is 2, 3, 4,
n is 0, 1, 2, 3, 4,
o is 0, 1, 2,
pis 0, 1, 2, 3, 4.

2. The compound of claim 1 according to formula (I') 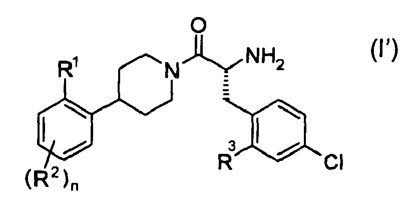 wherein R¹, R², R³ and n are as defined in claim 1.

3. The compound of claim 1 or 2, wherein
R² is Cl,
R³ is Cl.

4. The compound of any of claims 1 to 3, wherein
I is 3, and
m is 2 or 3.

5. The compound of any of claims 1 to 4 as medicament.

6. Use of the compound of any of claims 1 to 4 for the preparation of a medicament for the treatment or prophylaxis of disorders, diseases or conditions responsive to the inactivation of the melanocortin-4 receptor in a mammal.

7. Use according to claim 6 for the preparation of a medicament for the treatment or prophylaxis of cancer cachexia.

8. Use according to claim 6 for the preparation of a medicament for the treatment or prophylaxis of muscle wasting.

9. Use according to claim 6 for the preparation of a medicament for the treatment or prophylaxis of anorexia.

10. Use according to claim 6 for the preparation of a medicament for the treatment or prophylaxis of anxiety and/or depression.

11. A pharmaceutical composition comprising a compound of any of claims 1 to 4 and a pharmaceutically acceptable carrier.

12. A compound according to formula (II) 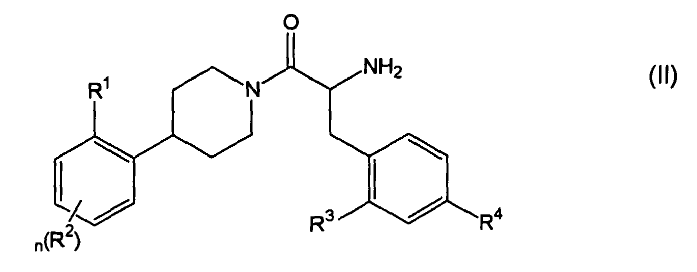 and enantiomers, diastereomers, tautomers, solvates and pharmaceutically acceptable salts thereof,
wherein
R¹ is -(CH₂)_{I}-T,
-O-(CH₂)ₘ-T,
T is NR⁵R⁶,
morpholine, 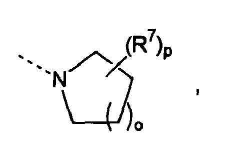 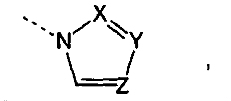
R⁵ and R⁶ are independently
C₁₋₆-alkyl,
C₂₋₆-alkenyl,
C₂₋₆-alkinyl,
C₂₋₆-alkylene-O-C₁₋₆-alkyl,
R⁷ is halogen,
CN,
OH,
C₁₋₆-alkyl optionally substituted with 1 to 3 substituents selected from halogen,
CN and OH,
O-C₁₋₆-alkyl optionally substituted with 1 to 3 substituents selected from halogen, CN and OH,
X is CH, N,
Y is CH, N,
Z is CH, N,
R² is F,
Cl,
methyl,
R³ is H,
F,
R⁴ is Cl.
F,
R⁴ is Cl,
F,
I is 3,4,
m is 2, 3, 4,
n is 0, 1, 2, 3, 4,
o is 0, 1, 2,
p is 0, 1, 2, 3, 4.

13. The compound of claim 12 according to formula (II') 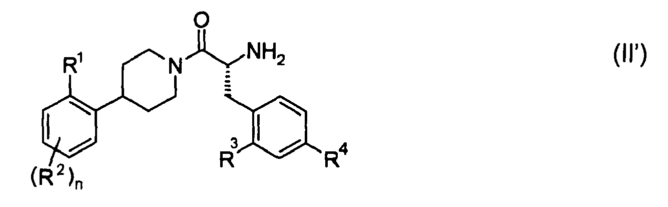 wherein R¹, R², R³, R⁴ and n are defined as in claim 12.

14. The compound of claim 12 or 13, wherein
R² is F or Cl, and
R³ is H.

15. The compound of any of claims 12 to 14, wherein
I is 3, and
m is 2 or 3.

16. The compound of any of claims 12 to 15 as medicament.

17. Use of the compound of any of claims 12 to 15 for the preparation of a medicament for the treatment or prophylaxis of disorders, diseases or conditions responsive to the activation of the melanocortin-4 receptor in a mammal.

18. Use according to claim 17 for the preparation of a medicament for the treatment or prophylaxis of obesity.

19. Use according to claim 17 for the preparation of a medicament for the treatment or prophylaxis of diabetes mellitus.

20. Use according to claim 17 for the preparation of a medicament for the treatment or prophylaxis of male or female sexual dysfunction.

21. Use according to claim 17 for the preparation of a medicament for the treatment or prophylaxis of erectile dysfunction.

22. A pharmaceutical composition comprising a compound of any of claims 12 to 15 and a pharmaceutically acceptable carrier.
